# EUROPEAN PATENT APPLICATION

(11) **EP 3 960 842 A1**
(43) Date of publication of application: **02.03.2022**
(21) Application number: 20794270.7
(22) Date of filing: 24.04.2020
(51) Int. Cl.: C12M 1/00, C12M 1/34, C12Q 1/04, C12Q 1/6844, C12Q 1/6851, C12Q 1/686, C12Q 1/6869

(54) **SELECTIVE DETECTION, COUNTING, AND GENOMIC ANALYSIS OF LIVING BACTERIUM-DERIVED NUCLEIC ACID ON SINGLE-ORGANISM BASIS**

(30) Priority: 26.04.2019 JP 2019085836; 06.11.2019 JP 2019201525
(71) Applicant: bitBiome, Inc., Tokyo 169-0051 (JP)
(72) Inventor: HOSOKAWA Masahito, Tokyo 169-8050 (JP); TAKEYAMA Haruko, Tokyo 169-8050 (JP); NISHIKAWA Yohei, Tokyo 169-8050 (JP); KOGAWA Masato, Tokyo 169-8050 (JP)
(74) Representative: Uexküll & Stolberg
(86) International application number: PCT/JP2020/017797
(87) International publication number: WO 2020/218557

(57) **Abstract**

The present disclosure provides a technique enabling analysis of cell viability information and nucleic acid information together for each single cell in a cell population when analyzing the composition of the cell population. The present disclosure discloses a method that is for analyzing the composition of a cell population and that comprises: a step for providing gel capsules in each of which a single cell of the cell population is encapsulated in a state of being labeled with a labeling agent, where the labeling agent distinctively labels a living cell and a dead cell; a step for amplifying a nucleic acid derived from each single cell of the cell population; a step for obtaining cell viability information for each single cell of the cell population; and a step for analyzing the amplified nucleic acid derived from each single cell of the cell population.

## Description

### [Technical Field]

The present disclosure relates to analysis of cell population composition and can be used in biological research, medicine, environment, healthcare, and other fields.

### [Background Art]

Selective detection of live bacteria from the environment is used for sanitation quality evaluation such as food inspection or seed testing and quality assurance of microbial inoculant or microbial formulations. For food inspection, target bacteria are specified, so that an approach of measuring the number of bacteria that grow under a certain condition by using a standard agar medium is commonly used for targets with an established culturing method in a laboratory. However, in view of the number of days required for culturing and complexity of culturing, a method of identification through observation of fluorescent stain of live and dead bacteria and the like has been proposed as an alternative technology. While the bacterial species of observed material can be inferred from determination based on an image for samples comprised of known species, it is challenging to identify the bacterial species composition for samples comprised of multiple species of unknown bacteria or similar bacteria. An approach based on detection of genes include an approach of specifically amplifying and detecting a sequence of the target bacteria, which uses PCR, qPCR, digital PCR, LAMP, or the like. Although gene detection-based approaches are highly sensitivity and are capable of processing multiple specimens, dead bacteria-derived nucleic acids are also simultaneously detected without specificity to live bacteria with conventional methods. Further, PCR and LAMP that detect only nucleic acids of live bacteria are compatible with only specific target species with a known sequence.

### [Summary of Invention]

### [Solution to Problem]

The present disclosure provides technologies such as a method of analyzing composition of a population of cells, and a reagent, composition, kit, and system used therein. One of the features of the technologies of the present disclosure is a step of obtaining information on whether a cell is alive or dead for each individual cell in the population of cells, a step of analyzing an amplified nucleic acid from each individual cell in the population of cells, or a reagent, composition, means, or the like used therein.

In one aspect, the present disclosure prepares amplified polynucleotides, which have been prepared in parallel for each single cell from advancing a reaction specifically for a nucleic acid derived from a live cell from a variety of microbes, and executes comprehensive reading of total live cell count or a genetic sequence that identifies a microbe species to provide live cell derived cell count or sequence information from a cell population in which live cells and dead cells coexist. The basic concept is to first label dead cells, amplify nucleic acids, then label amplified nucleic acids derived from a live cell, and specifically detect a nucleic acid derived from a live cell.

Examples of the embodiments of the present disclosure include the following.
(Item A)
   A method of analyzing composition of a population of cells, comprising:
   a step of obtaining information on whether a cell is alive or dead for each individual cell in the population of cells; and
   a step of analyzing an amplified nucleic acid derived from each individual cell in the population of cells.
(Item AA)
   A method of providing information on a nucleic acid of a live cell and a dead cell for each single cell in a population of cells, comprising:
   a step of obtaining information on whether a cell is alive or dead for each individual cell in the population of cells; and
   a step of analyzing an amplified nucleic acid derived from each individual cell in the population of cells.
(Item 1)
   A method of analyzing composition of a population of cells, comprising:
   a step of providing gel capsules each encapsulating an individual cell of the population of cells being labeled with a labeling agent, wherein the labeling agent differentially labels a live cell and a dead cell as information on whether a cell is alive or dead for each individual cell in the population of cells;
   a step of amplifying a nucleic acid derived from each individual cell in the population of cells;
   a step of obtaining information on whether a cell is alive or dead for each individual cell in the population of cells; and
   a step of analyzing an amplified nucleic acid derived from each individual cell in the population of cells.
(Item 2)
   The method of any one of the preceding items, wherein the step of providing gel capsules each encapsulating an individual cell of the population of cells comprises:
   a step of adding the labeling agent to the population of cells; and
   a step of individually encapsulating each of cells of the population of cells in the gel capsule.
(Item 3)
   The method of any one of the preceding items, wherein the step of providing gel capsules each encapsulating an individual cell of the population of cells comprises:
   a step of individually encapsulating each of the cells of the population of cells in the gel capsule; and
   a step of adding the labeling agent to the gel capsules.
(Item 4)
   The method of any one of the preceding items, wherein the analysis of the amplified nucleic acid is performed on a nucleic acid derived from a live cell.
(Item 5)
   The method of any one of the preceding items, wherein the labeling agent specifically labels a dead cell or specifically labels a live cell.
(Item 6)
   The method of any one of the preceding items, further comprising a step of adding a nucleic acid degradation promoting agent for promoting degradation of a nucleic acid derived from a dead organism to the population of cells or the gel capsules.
(Item 7)
   The method of any one of the preceding items, wherein the nucleic acid degradation promoting agent is a topoisomerase inhibitor.
(Item 8)
   The method of any one of the preceding items, wherein the nucleic acid degradation promoting agent comprises one or more selected from Ciprofloxacin (CPFX), Camptothecin (CPT), Etoposide (ETP), and Amsacrine (m-AMSA).
(Item 9)
   The method of any one of the preceding items, wherein the labeling agent is a substance that penetrates through a membrane of a dead cell.
(Item 10)
   The method of any one of the preceding items, wherein the labeling agent is a substance that penetrates through a membrane of a dead cell and binds to a nucleic acid.
(Item 11)
   The method of any one of the preceding items, wherein the labeling agent is a substance that inhibits amplification of a bound nucleic acid.
(Item 12)
   The method of any one of the preceding items, wherein the labeling agent comprises one or more selected from ethidium monoazide (EMA), psoralen, 4'-aminomethyl-4,5'-dimethylisopsoralen [4'-AMDMIP], and propidium monoazide (PMA).
(Item 13)
   The method of any one of the preceding items, wherein the information on whether a cell is alive or dead for each individual cell is obtained from the presence/absence of amplification of a nucleic acid derived from each individual cell.
(Item 14)
   The method of any one of the preceding items, wherein the presence/absence of amplification of a nucleic acid derived from each individual cell is detected using a nucleic acid binding fluorescent substance capable of detecting amplification of a nucleic acid.
(Item 15)
   The method of any one of the preceding items, wherein the nucleic acid binding fluorescent substance comprises one or more selected from EvaGreen, SYBR Green, SYBR Gold, SYTO9, SYTO10, Hoechst 33342, 4',6-diamidino-2-phenylindole, Acridine Orange (AO), Promidium iodide, and Ethidium homodimer-2.
(Item 16)
   The method of any one of the preceding items, wherein the step of encapsulating cells of the population of cells each individually in the gel capsules comprises:
   a step of individually encapsulating each of the cells of the population of cells in a liquid droplet; and
   a step of gelating the liquid droplet to produce gel capsule.
(Item 17)
   The method of any one of the preceding items, wherein a suspension of the cells is allowed to flow in a microchannel, and the suspension is sheared with oil to prepare the liquid droplets encapsulating the cells.
(Item 18)
   The method of any one of the preceding items, wherein the gel capsule is formed from agarose, acrylamide, PEG, gelatin, sodium alginate, Matrigel, collagen, or photocurable resin.
(Item 19)
   The method of any one of the preceding items, wherein the gel capsules are hydrogel capsules.
(Item 20)
   The method of any one of the preceding items, wherein the step of amplifying a nucleic acid derived from each individual cell in the population of cells comprises:
   a step of immersing the gel capsules in one or more lysis reagents to lyse the cells, wherein a genomic DNA of the cells or a polynucleotide comprising said portion elutes out into the gel capsules and is retained in the gel capsules; and
   a step of contacting the polynucleotide with an amplification reagent to amplify the polynucleotide in a gel capsule.
(Item 21)
   The method of any one of the preceding items, further comprising a step of removing a contaminant from the gel capsules.
(Item 22)
   The method of any one of the preceding items, wherein at least one is selected as the lysis reagent from the group consisting of lysozyme, labiase, Yatalase, achromopeptidase, protease, nuclease, zymolyase, chitinase, lysostaphin, mutanolysin, sodium dodecyl sulfate, sodium lauryl sulfate, potassium hydroxide, sodium hydroxide, phenol, chloroform, guanidine hydrochloride, urea, 2-mercaptoethanol, dithiothreitol, TCEP-HCl, sodium cholate, sodium deoxycholate, Triton X-100, Triton X-114, NP-40, Brij-35, Brij-58, Tween 20, Tween 80, octyl glucoside, octyl thioglucoside, CHAPS, CHAPSO, dodecyl-p-D-maltoside, Nonidet P-40, and Zwittergent 3-12.
(Item 23)
   The method of any one of the preceding items, further comprising a step of selecting a sample comprising the amplified nucleic acid to be analyzed from samples comprising an amplified nucleic acid from the each individual cell.
(Item 24)
   The method of any one of the preceding items, wherein the analysis of the amplified nucleic acid comprises identifying a type of the each individual cell.
(Item 25)
   The method of any one of items 1 to 24, wherein the analysis of the amplified nucleic acid comprises a step of detecting a nucleic acid having a specific sequence in samples comprising an amplified nucleic acid from the each individual cell.
(Item 26)
   The method of any one of the preceding items, wherein the step of detecting a nucleic acid having a specific sequence comprises amplifying a nucleic acid having a specific sequence and sequencing the nucleic acid.
(Item 27)
   The method of any one of the preceding items, wherein the analysis of composition of a population of cells comprises identifying an absolute number of various cells in the population of cells.
(Item 28)
   The method of any one of the preceding items, wherein the analysis of composition of a population of cells comprises identifying an absolute number of various cells in live cells in the population of cells.
(Item 29)
   The method of any one of the preceding items, further comprising a step of obtaining genomic sequence data of the each individual cell from a sample comprising an amplified nucleic acid derived from each individual cell in the population of cells.
(Item 30)
   The method of any one of the preceding items, wherein the population of cells is a microbiota.
(Item 31)
   The method of any one of the preceding items for evaluating quality of an FMT specimen.
(Item 32)
   The method of any one of the preceding items for evaluating sanitary quality of a food product.
(Item 33)
   The method of any one of the preceding items for evaluating the presence of a pathogenic bacterium in an environment.
(Item 34)
   The method of any one of the preceding items, wherein the population of cells is a microbial formulation.
(Item 35)
   The method of any one of the preceding items for evaluating quality of a microbial formulation.
(Item 36)
   The method of any one of the preceding items for evaluating quality of a microbe derived animal feed, a fermented food product, or a microbe containing supplement.
(Item 37)
   The method of any one of the preceding items for profiling live bacteria in activated sludge.
(Item 38)
   The method of any one of the preceding items for evaluating the presence of a live microbe in an environment.
(Item 39)
   The method of any one of the preceding items for evaluating an effect or spectrum of an antibacterial drug.
(Item 40)
   The method of any one of the preceding items for evaluating a sterilization method.
(Item 41)
   The method of any one of the preceding items for profiling live bacteria in a biofilm.
(Item B1)
   A method comprising:
   a step of adding ethidium monoazide (EMA) or propidium monoazide (PMA) to a population of cells and irradiating light;
   a step of individually encapsulating each of the cells of the population of cells in a liquid droplet;
   a step of gelating the liquid droplet to produce gel capsule;
   a step of immersing the gel capsule in one or more lysis reagents to lyse the cells, wherein a genomic DNA of the cells or a polynucleotide comprising said portion elutes out into the gel capsule;
   a step of removing a contaminant from the gel capsule;
   a step of contacting the polynucleotide with an amplification reagent to amplify the polynucleotide in the gel capsule, wherein a polynucleotide bound to EMA or PMA is not amplified;
   a step of detecting an amplified polynucleotide;
   a step of selecting gel capsules comprising a sample derived from a live bacterium;
   a step of counting gel capsules comprising a sample derived from a live bacterium; and
   a step of analyzing a polynucleotide amplified in a gel capsule comprising a sample derived from a live bacterium.
(Item B2)
   A method comprising:
   a step of individually encapsulating each of the cells of the population of cells in a liquid droplet;
   a step of gelating the liquid droplet to produce gel capsule;
   a step of adding EMA or PMA to the gel capsule and irradiating light;
   a step of washing the gel capsule;
   a step of immersing the gel capsule in one or more lysis reagents to lyse the cells, wherein a genomic DNA of the cells or a polynucleotide comprising said portion elutes out into the gel capsule;
   a step of removing a contaminant from the gel capsule;
   a step of contacting the polynucleotide with an amplification reagent to amplify the polynucleotide in the gel capsule, wherein a polynucleotide bound to EMA or PMA is not amplified;
   a step of detecting an amplified polynucleotide;
   a step of selecting gel capsules comprising a sample derived from a live bacterium;
   a step of counting gel capsules comprising a sample derived from a live bacterium; and
   a step of analyzing a polynucleotide amplified in a gel capsule comprising a sample derived from a live bacterium.
(Item C)
   A system for analyzing composition of a population of cells, comprising:
   means for obtaining information on whether a cell is alive or dead for each individual cell in the population of cells; and
   means for analyzing an amplified nucleic acid derived from each individual cell in the population of cells.
(Item C-1)
   The system of the preceding item, comprising a feature of any one or more of the preceding items.
(Item C1)
   A system or kit for analyzing composition of a population of cells, comprising:
   a labeling agent for differentially labeling a live cell and a dead cell;
   means for encapsulating the cells in gel capsules;
   means for amplifying a nucleic acid; and
   means for analyzing the nucleic acid.
(Item C1-1)
   The system or kit of the preceding item, comprising a feature of any one or more of the preceding items.
(Item C2)
   A composition for use in a method of analyzing composition of a population of cells, comprising a labeling agent for differentially labeling a live cell and a dead cell, wherein the method comprises: a step of obtaining information on whether a cell is alive or dead for each individual cell in the population of cells; and a step of analyzing an amplified nucleic acid derived from each individual cell in the population of cells.
(Item C2-1)
   The composition of the preceding item, comprising a feature of any one or more of the preceding items.
(Item C3)
   A composition for use in a method of individually encapsulating each of the cells of a population of cells in a gel capsule for analysis, comprising a labeling agent for differentially labeling a live cell and a dead cell.
(Item C3-1)
   The composition of the preceding item, comprising a feature of any one or more of the preceding items.
(Item C4)
   The system, kit, or composition of any one of the preceding items, wherein the analysis of the amplified nucleic acid is performed on a nucleic acid derived from a live cell.
(Item C5)
   The system, kit, or composition of any one of the preceding items, wherein the labeling agent specifically labels a dead cell or specifically labels a live cell.
(Item C6)
   The system, kit, or composition of any one of the preceding items, further comprising a nucleic acid degradation promoting agent for promoting degradation of a nucleic acid derived from a dead bacterium, which is added to the population of cells or the gel capsules.
(Item C7)
   The system, kit, or composition of any one of the preceding items, wherein the nucleic acid degradation promoting agent is a topoisomerase inhibitor.
(Item C8)
   The system, kit, or composition of any one of the preceding items, wherein the nucleic acid degradation promoting agent comprises one or more selected from Ciprofloxacin (CPFX), Camptothecin (CPT), Etoposide (ETP), and Amsacrine (m-AMSA).
(Item C9)
   The system, kit, or composition of any one of the preceding items, wherein the labeling agent is a substance that penetrates through a membrane of a dead cell.
(Item C10)
   The system, kit, or composition of any one of the preceding items, wherein the labeling agent is a substance that penetrates through a membrane of a dead cell and binds to a nucleic acid.
(Item C11)
   The system, kit, or composition of any one of the preceding items, wherein the labeling agent is a substance that inhibits amplification of a bound nucleic acid.
(Item C12)
   The system, kit, or composition of any one of the preceding items, wherein the labeling agent comprises one or more selected from ethidium monoazide (EMA), psoralen, 4'-aminomethyl-4,5'-dimethylisopsoralen [4'-AMDMIP], and propidium monoazide (PMA).
(Item C13)
   The system, kit, or composition of any one of the preceding items, wherein the information on whether a cell is alive or dead for each individual cell is obtained from the presence/absence of amplification of a nucleic acid derived from each individual cell.
(Item C14)
   The system, kit, or composition of any one of the preceding items, further comprising means for detecting the presence/absence of amplification of a nucleic acid derived from each individual cell (e.g., a nucleic acid binding fluorescent substance capable of detecting amplification of a nucleic acid).
(Item C15)
   The system, kit, or composition of any one of the preceding items, wherein the nucleic acid binding fluorescent substance comprises one or more selected from EvaGreen, SYBR Green, SYBR Gold, SYTO9, SYTO10, Hoechst 33342, 4',6-diamidino-2-phenylindole, Acridine Orange (AO), Promidium iodide, and Ethidium homodimer-2.
(Item C16)
   The system, kit, or composition of any one of the preceding items, further comprising:
   means for individually encapsulating each of cells of a population of cells in a liquid droplet or a liquid droplet preparation unit for individually encapsulating each of cells of a population of cells in a liquid droplet; and
   means for gelating liquid droplets to produce gel capsules or a gel capsule producing unit for gelating liquid droplet to produce gel capsules.
(Item C17)
   The system, kit, or composition of any one of the preceding items, configured so that a suspension of the cells is allowed to flow in a microchannel, and the suspension is sheared with oil to prepare the liquid droplets encapsulating the cells.
(Item C18)
   The system, kit, or composition of any one of the preceding items, wherein the gel capsules are formed from agarose, acrylamide, PEG, gelatin, sodium alginate, Matrigel, collagen, or photocurable resin.
(Item C19)
   The system, kit, or composition of any one of the preceding items, wherein the gel capsules are hydrogel capsules.
(Item C20)
   The system, kit, or composition of any one of the preceding items, further comprising:
   means for lysing cells or a lysis reagent immersing unit for immersing gel capsules in a lysis reagent, configured so that a genomic DNA of the cells or a polynucleotide comprising said portion elutes out into the gel capsules and is retained in the gel capsules; and
   means for amplifying a polynucleotide in a gel capsule or an amplification reagent immersing unit for immersing a gel capsule in an amplification reagent.
(Item C21)
   The system, kit, or composition of any one of the preceding items, further comprising means for removing a contaminant from the gel capsules or a removing unit for removing a contaminant from gel capsules.
(Item C22)
   The system, kit, or composition of any one of the preceding items, wherein at least one is selected as the lysis reagent from the group consisting of lysozyme, labiase, Yatalase, achromopeptidase, protease, nuclease, zymolyase, chitinase, lysostaphin, mutanolysin, sodium dodecyl sulfate, sodium lauryl sulfate, potassium hydroxide, sodium hydroxide, phenol, chloroform, guanidine hydrochloride, urea, 2-mercaptoethanol, dithiothreitol, TCEP-HCl, sodium cholate, sodium deoxycholate, Triton X-100, Triton X-114, NP-40, Brij-35, Brij-58, Tween 20, Tween 80, octyl glucoside, octyl thioglucoside, CHAPS, CHAPSO, dodecyl-p-D-maltoside, Nonidet P-40, and Zwittergent 3-12.
(Item C23)
   The system, kit, or composition of any one of the preceding items, further comprising means for selecting a sample comprising the amplified nucleic acid to be analyzed from samples comprising the amplified nucleic acid from the each individual cell or a screening unit for screening a gel capsule and housing the gel capsule in a container.
(Item C24)
   The system, kit, or composition of any one of the preceding items, wherein the analysis of the amplified nucleic acid comprises identifying a type of the each individual cell.
(Item C25)
   The system, kit, or composition of any one of the preceding item, wherein the analysis of the amplified nucleic acid comprises a step of detecting a nucleic acid having a specific sequence in samples comprising the amplified nucleic acid from the each individual cell.
(Item C26)
   The method of item 25, wherein the step of detecting a nucleic acid having a specific sequence comprises amplifying a nucleic acid having a specific sequence and sequencing the nucleic acid.
(Item C27)
   The system, kit, or composition of any one of the preceding items, wherein the analysis of composition of a population of cells comprises identifying an absolute number of various cells in the population of cells.
(Item C28)
   The system, kit, or composition of any one of the preceding items, wherein the analysis of composition of a population of cells comprises identifying an absolute number of various cells in live cells in the population of cells.
(Item C29)
   The system, kit, or composition of any one of the preceding items, further comprising a step of obtaining genomic sequence data of the each individual cell from a sample comprising an amplified nucleic acid derived from each individual cell in the population of cells.
(Item C30)
   The system, kit, or composition of any one of the preceding items, wherein the population of cells is a microbiota.
(Item C31)
   The system, kit, or composition of any one of the preceding items for evaluating quality of an FMT specimen.
(Item C32)
   The system, kit, or composition of any one of the preceding items for evaluating sanitary quality of a food product.
(Item C33)
   The system, kit, or composition of any one of the preceding items for evaluating the presence of a pathogenic bacterium in an environment.
(Item C34)
   The system, kit, or composition of any one of the preceding items, wherein the population of cells is a microbial formulation.
(Item C35)
   The system, kit, or composition of any one of the preceding items for evaluating quality of a microbial formulation.
(Item C36)
   The system, kit, or composition of any one of the preceding items for evaluating quality of a microbe derived animal feed, a fermented food product, or a microbe containing supplement.
(Item C37)
   The system, kit, or composition of any one of the preceding items for profiling live bacteria in activated sludge.
(Item C38)
   The system, kit, or composition of any one of the preceding items for evaluating the presence of a live microbe in an environment.
(Item C39)
   The system, kit, or composition of any one of the preceding items for evaluating an effect or spectrum of an antibacterial drug.
(Item C40)
   The system, kit, or composition of any one of the preceding items for evaluating a sterilization method.
(Item C41)
   The system, kit, or compositino of any one of the preceding items for profiling live bacteria in a biofilm.
(Item D1)
   A system or kit comprising:
   ethidium monoazide (EMA) or propidium monoazide (PMA) (a reagent for distinguishing whether a cell is alive or dead);
   light irradiation means;
   means for individually encapsulating each of cells in a liquid droplet;
   means for gelating the liquid droplets to produce gel capsules;
   a lysis reagent for the gel capsules, wherein the lysis reagent is added and used so that a genomic DNA of the cells or a polynucleotide comprising said portion elutes out into the gel capsules;
   means for removing a contaminant from the gel capsules;
   an amplification reagent for a polynucleotide, wherein the amplification reagent is used so that the polynucleotide is contacted with the amplification reagent to amplify the polynucleotide in the gel capsules, but a polynucleotide bound to EMA or PMA is not amplified;
   means for detecting a polynucleotide;
   means for selecting gel capsules comprising a sample derived from a live bacterium;
   means for counting gel capsules comprising a sample derived from a live bacterium; and
   means for analyzing a polynucleotide amplified in a gel capsule comprising a sample derived from a live bacterium.
(Item D2)
   A composition for use in a method of analyzing composition of a population of cells, comprising a labeling agent for differentially labeling a live cell and a dead cell (e.g., ethidium monoazide (EMA) or propidium monoazide (PMA)), wherein the method comprises a step of obtaining information on whether a cell is alive or dead for each individual cell in the population of cells; and a step of analyzing an amplified nucleic acid derived from each individual cell in the population of cells.

The present disclosure is intended so that one or more of the features described above can be provided not only as the explicitly disclosed combinations, but also as other combinations thereof. Additional embodiments and advantages of the present disclosure are recognized by those skilled in the art by reading and understanding the following detailed description as needed.

### [Advantageous Effects of Invention]

Distinction of live cells from dead cells is required in various biological studies. Analysis in a mixed sample of multiple cell species was particularly challenging. The present disclosure can evaluate the live bacteria count of bacteria that are present as live cells in a sample, regardless of whether bacteria can be cultured or the presence/absence of genetic sequence information, and is characterized by the ability to obtain whole genomic sequence information. Due to the progression in degradation of DNA derived from dead bacteria, such DNA can be noise in genome analysis at a single organism level. Thus, such distinction can also be effective in terms of obtaining genome sequence information by limiting a sample to live bacteria in advance. Quality evaluation of a microbiota utilized in fecal transplant, microbial formulation, or the like is not limited to known sequences. Versatility is high in that diverse subjects can be evaluated by the same analysis format.

### [Brief Description of Drawings]

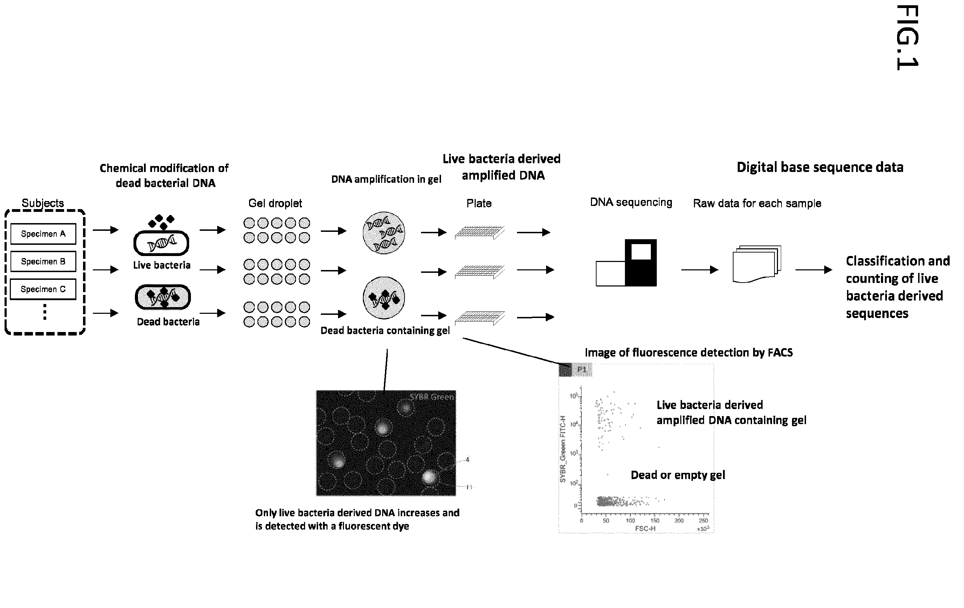

[Figure 1] Figure **1** is a schematic diagram exemplifying the flow of selective detection, counting, and genome analysis of DNA derived from live cells. As a preliminary sample processing, dead cell DNA is chemically modified into a form that does not replicate by an enzymatic reaction. Both live cells and dead cells are lysed in gel to purify DNA. However, only DNA derived from live cells increases upon DNA amplification. The total live cell count is measured when collecting the amplified live cell DNA-gel. Subsequently, they are individually sequenced to classify and count sequences derived from live cells.

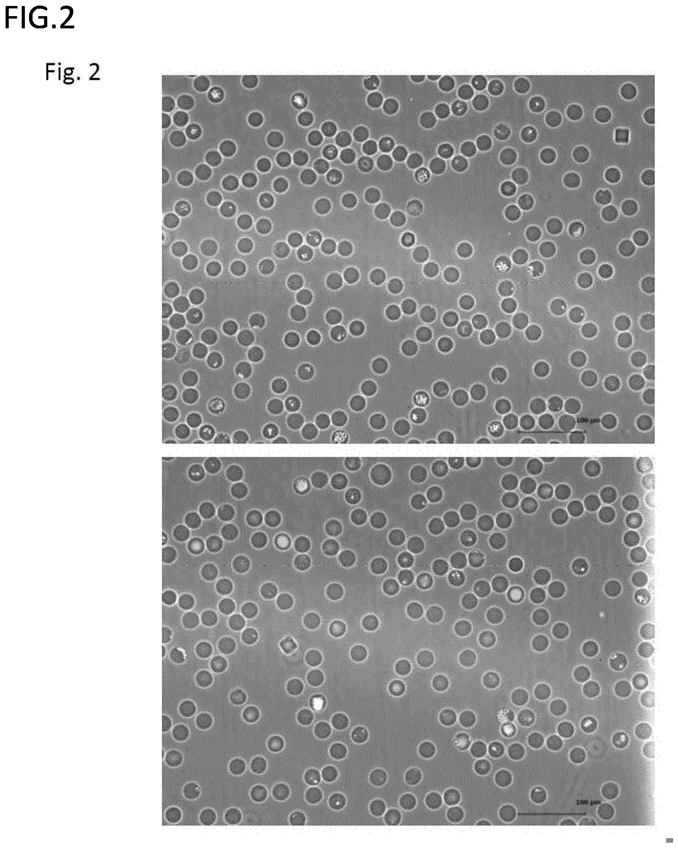

[Figure 2] Figure **2** shows an observed image after heating and amplifying a polynucleotide of an EMA treated E. coli-containing gel capsule (top row: with EMA, bottom row: without EMA). EMA treatment suppressed polynucleotide amplification, and fluorescence (green bright spots) is no longer visible in heated and sterilized samples.

### [Description of Embodiments]

The present disclosure is described hereinafter while showing the best mode thereof. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. The terms used herein should also be understood as being used in the meaning that is commonly used in the art, unless specifically noted otherwise. Thus, unless defined otherwise, all terminologies and scientific technical terms that are used herein have the same meaning as the general understanding of those skilled in the art to which the disclosure pertains. In case of a contradiction, the present specification (including the definitions) takes precedence.

### (Definitions, etc.)

The definitions of the terms and/or basic technical matters especially used herein are described hereinafter when appropriate.

As used herein, "cell" refers to any particle containing a molecule with genetic information, which can be replicated (regardless of whether this is possible alone). The "cell" as used herein encompasses anything that can be distinguished as to whether it is alive or dead, thus, encompassing cells of unicellular organisms, bacteria, cells derived from a multicellular organism, fungi, cells that are symbiotic with a virus, and the like.

As used herein, "biomolecule" refers to a molecule of any organism or virus. A biomolecule can include a nucleic acid, protein, sugar chain, lipid, or the like. As used herein, "analog of biomolecule" refers to a naturally-occurring or non-naturally-occurring variant of a biomolecule. An analog of a biomolecule can include a modified nucleic acid, modified amino acid, modified lipid, modified sugar chain, or the like.

As used herein, "population" refers to a collection comprising two or more cells.

As used herein, "subpopulation" refers to a part of a population with fewer number of cells than the population.

As used herein, "gel" refers to a colloidal solution (sol) wherein a polymeric substance or colloidal particles form a mesh structure as a whole due to the interaction thereof, without fluidity while containing a large quantity of a liquid phase that is a solvent or dispersion medium. As used herein, "gelation" refers to changing a solution into a state of "gel".

As used herein, "gel capsule" refers to a gel-like microparticulate construct that can retain a cell or cell-like construct therein.

As used herein, "genetic analysis" refers to studying the state of a nucleic acid (DNA, RNA, or the like) in a biological sample. In one embodiment, genetic analysis includes those that utilize a nucleic acid amplification reaction. Examples of genetic analysis include, in addition thereto, sequencing, genotyping/polymorphism analysis (SNP analysis, copy number variation, restriction fragment length polymorphism, repeat number polymorphism), expression analysis, Quenching Probe (Q-Probe), SYBR green method, melt curve analysis, real-time PCR, quantitative RT-PCR, digital PCR, and the like.

As used herein, "single cell level" refers to processing of genetic information contained in a single cell distinctly from genetic information contained in other cells. Thus, single cell level can also be expressed by a term such as "for each (-) cell". For example, when a polynucleotide is amplified at a "single cell level", a polynucleotide in a cell and a polynucleotide in another cell are each amplified in distinguishable states.

As used herein, "single cell analysis" refers to analysis of genetic information contained in a single cell distinctly from genetic information contained in other cells.

As used herein, "nucleic acid information" refers to information on a nucleic acid contained in a single cell, including the presence/absence of a specific genetic sequence, yield of a specific gene, or total nucleic acid yield.

As used herein, "identity" refers to sequence similarity between two nucleic acid molecules. Identity can be determined by comparing positions in each sequence that can be aligned for comparison.

As used herein, "microbiota" refers to the entire population of microbes that are present in a certain physical range. Examples of certain physical range include certain ranges in the intestine, skin, mouth, nasal cavity, or vagina of an individual, and body of water or soil in an environment, organism surface, inside of an organism, and the like. A microbiota can be a population of microbes classified into multiple species such as a combination of fungi, bacteria, archaea, single cell animals, viruses, individual cells of multicellular organisms, or the like. Microbe of some classifications can be selected out as a population. An example of microbiota includes a bacterial flora.

As used herein, "composition" of a cell population refers to information regarding the species contained in the cell population or the amount of each contained species. Composition also encompasses information regarding whether some microbe species in a cell population is included, and the amount thereof.

As used herein, "to label" or "labeling" refers to enabling distinction based on some type of feature. Representative examples include enabling distinction from having a substance specific to a cell having some type of feature bound by "labeling". Meanwhile, enabling distinction from not having a substance bound to a cell having some type of a feature can also be referred to as "labeling".

As used herein, "labeling agent" refers to any substance that can be used to distinguish a cell or a nucleic acid based on some type of a feature. Representative examples thereof include substances that can be used for differentially labeling and distinguishing live cells from dead cells.

### (Summary of the present disclosure)

The summary of the present disclosure is described below with reference to embodiments. Embodiments described below are provided to facilitate the understanding of the present disclosure. It is understood that the scope of the present disclosure should not be limited to the following descriptions. Thus, it is apparent that those skilled in the art can make appropriate modifications within the scope of the present disclosure by referring to the descriptions herein. It is also understood that the following embodiments can be used independently or as a combination thereof.

The present disclosure can provide a method of analyzing composition of a population of cells. The method can comprise a step of providing gel capsules each encapsulating an individual cell of the population of cells being labeled with a labeling agent. The labeling agent can differentially label a live cell and a dead cell. The labeling agent can differentially label a live cell and a dead cell as information on whether a cell is alive or dead for each individual cell in the population of cells. The method can comprise a step of amplifying a nucleic acid derived from each individual cell in the population of cells; a step of obtaining information on whether a cell is alive or dead for each individual cell in the population of cells; and a step of analyzing an amplified nucleic acid derived from each individual cell in the population of cells. The present disclosure also provides a method of providing information on a nucleic acid of a live cell and a dead cell for each single cell in a population of cells. This method can comprise a step of obtaining information on whether a cell is alive or dead for each individual cell in the population of cells; and a step of analyzing an amplified nucleic acid derived from each individual cell in the population of cells.

The present disclosure can also provide a system, kit, composition, or the like for analyzing composition of a population of cells. The system, kit, or composition can comprise a labeling agent for labeling a cell, and means for providing gel capsules each encapsulating an individual cell of the cells, or a composition therefor. The labeling agent can differentially label a live cell and a dead cell. The system, kit, or composition comprises a composition, reagent, or means for amplifying a nucleic acid, wherein the amplification of a nucleic acid is used to amplify a nucleic acid from each individual cell in the population of cells and can obtain information on whether a cell is alive or dead for each individual cell in the population of cells. The system, kit, or composition comprises means, reagent, kit, system, or the like for analyzing a nucleic acid, and analyzes an amplified nucleic acid derived from each individual cell in the population of cells. The present disclosure also provides a system, kit, composition, apparatus, agent, or the like for providing information on a nucleic acid of a live cell and a dead cell for each single cell in a population of cells. The system, kit, composition, apparatus, agent, or the like can comprise means, agent, or the like for obtaining information on whether a cell is alive or dead for each individual cell in a population of cells and means, agent, or the like for analyzing an amplified nucleic acid from each individual cell in a population of cells.

The step of labeling by adding a labeling agent can be performed before or after encapsulating a cell in a gel capsule. If labeling is performed by adding a labeling agent after encapsulating a cell in a gel capsule, an effect such as washing off an excess labeling agent via the capsule can be expected.

The step of providing gel capsules each encapsulating an individual cell of the population of cells can comprise a step of adding a labeling agent to the population of cells and a step of individually encapsulating each of cells of the population of cells in the gel capsule. The step of providing gel capsules each encapsulating an individual cell of the population of cells can comprises a step of individually encapsulating each of cells of the population of cells in the gel capsule; and a step of adding the labeling agent to the gel capsules.

Analysis of an amplified nucleic acid can be performed on a nucleic acid derived from a live cell by differential labeling with a labeling agent. If a cell in a sample is analyzed on a nucleic acid basis (e.g., metagenomics), information on a nucleic acid derived from a live cell and information on a nucleic acid derived from a dead cell cannot be distinguished, so that it is unclear whether information on a cell that is actually alive is obtained. When analyzing a cell in a sample at a single cell level, a cell that is in a state of a dead cell may be detected as a single cell in some cases. There are dead cells that have completely lost the morphology as a cell with components floating in a sample and dead cells that have maintained their shape. Conventional art could not remove dead cells of the latter, so that low quality genome amplification would be performed in some cases. The method of the present disclosure can prevent such low quality genome amplification by performing analysis on only nucleic acids derived from live cells. If information can be obtained as to which cells the live cells (e.g., live bacteria) are, the percentage of dead cells (e.g., dead bacteria) can be found from the difference in results of analysis using an approach that does not use this method.

PCR and LAMP that specifically amplify and detect a nucleic acid of a live cell have been reported (Nogva et al., 2003; Soejima et al., 2008), but such methods are only compatible with specific species with a known sequence. The principle of such methods is to detect only live bacterial DNA by treating and decomposing dead bacterial DNA with Ethidium monoazide (EMA), which efficiently modifies the DNA of dead cells, Topoisomerase poison Ciprofloxacin (CPFX), Camptothecin (CPT), or Etoposide (ETP), which inhibits the action of topoisomerase that is required for DNA replication, or the like.

In the present disclosure, the step of individually encapsulating each of cells of the population of cells in the gel capsule can comprise a step of individually encapsulating each of cells of the population of cells in a liquid droplet; and a step of gelating the liquid droplets to produce gel capsules.

### (Obtaining information on whether a cell is alive or dead)

In the present disclosure, a labeling agent can differentially label a live cell and a dead cell. A labeling agent can specifically label a dead cell, specifically label a live cell, or label cells so that live cells can be distinguished from dead cells. A labeling agent is not particularly limited, as long as the difference in properties such as the morphologies of dead cells and live cells can be utilized. Typically, a labeling agent is a substance that penetrates through a membrane of a dead cell. Since the integrity of the cell membrane (and/or cell wall) of dead cells is more compromised relative to live cells, a substance that penetrates through a cell membrane of dead cells but not through a cell membrane of live cells can be utilized as a labeling agent. Information on whether a cell is alive or dead for each individual cell can be obtained by detecting the presence/absence of such a labeling agent in a subsequent step. A labeling agent can be an agent that inhibits amplification of a nucleic acid or an agent that induces a change in charge or signal such as staining, fluorescence, luminescence, Raman scattering, or radiation.

A labeling agent can also be a substance that penetrates through a membrane of a dead cell and binds to a nucleic acid. A labeling agent can bind to a nucleic acid by, for example, covalent bond to or crosslinking reaction with a nucleic acid, or intercalation into the spiral of a nucleic acid. Information on whether a cell is alive or dead for each individual cell can be obtained from detecting the presence/absence of such a bond in a subsequent step. A representative labeling agent can be a substance that inhibits amplification of a bound nucleic acid. Information on whether a cell is alive or dead for each individual cell can be obtained from the presence/absence of amplification of a nucleic acid derived from each individual cell in a subsequent step. Examples of labeling agents include ethidium monoazide (EMA), psoralen, 4'-aminomethyl-4,5'-dimethylisopsoralen [4'-AMDMIP], propidium monoazide (PMA), and the like. In the present disclosure, one or more selected therefrom can be comprised as a labeling agent.

A nucleic acid degradation promoting agent for promoting degradation of a nucleic acid derived from a dead bacterium can be added in addition to adding a labeling agent. Examples of nucleic acid degradation promoting agent include topoisomerase inhibitors which inhibit the action of topoisomerase that is required for DNA replication, gyrase inhibitors which inhibit the action of gyrase that is required for DNA replication in some microbes, and the like. In the present disclosure, one or more selected therefrom can be comprised as a nucleic acid degradation promoting agent. Examples of nucleic acid degradation promoting agents include Ciprofloxacin (CPFX), Camptothecin (CPT), Etoposide (ETP), Amsacrine (m-AMSA), and the like. In the present disclosure, one or more selected therefrom can be comprised as a nucleic acid degradation promoting agent.

If a substance that inhibits amplification of a nucleic acid is used as a labeling agent, information on whether a cell is alive or dead for each individual cell can be obtained from the presence/absence of amplification of a nucleic acid derived from each individual cell. The presence/absence of amplification of a nucleic acid can be detected using, for example, a nucleic acid binding fluorescent substance that is capable of detecting amplification of a nucleic acid. Examples of nucleic acid binding fluorescent substances include EvaGreen, SYBR Green, SYBR Gold, SYTO9, SYTO10, Hoechst 33342, 4',6-diamidino-2-phenylindole, Acridine Orange (AO), Promidium iodide, Ethidium homodimer-2, and the like. In the present disclosure, one or more selected therefrom can be comprised as a nucleic acid binding fluorescent substance.

### (Single cell analysis)

One of the features of one aspect of the present disclosure is in obtaining information (information on whether a cell is alive or dead and nucleic acid information) for each individual cell in a method of analyzing composition of a cell population. This can be materialized by analyzing each individual cell by a method described in detail below. Although the amount of a nucleic acid with a certain sequence can be identified from information on nucleic acids derived from multiple cells, there is a difference in the number of copies for each type of cell (e.g., microbe species), so that the absolute amount of a specific type of cells cannot be measured. However, the absolute amount of a specific type of cells can be measured based on information on a nucleic acid derived from each individual cell. The present disclosure can also measure the absolute amount, number, ratio, or the like for live cells among the specific type of cells. In particular, the amount of nucleic acid derived from a microbe per cell is low so that an amplification reaction is commonly used even if nucleic acids derived from multiple cells are analyzed in a mixed state. Meanwhile, it is known that a bias results in the degree of amplification for each sequence depending on the GC content or the like even if nucleic acids are amplified as a whole by using a random primer or the like. If amplification is biased, measurement of the relative amount of a specific species of microbe within a microbiota would be difficult.

When analyzing cells by each individual cell, cells of a population of cells can be each individually encapsulated in gel capsules. The step of individually encapsulating each of cells of the population of cells in the gel capsule comprises: a step of individually encapsulating each of cells of the population of cells in a liquid droplet; and a step of gelating the liquid droplets to produce gel capsules.

Thus, the system, kit, composition, or the like of the present disclosure can comprise means, composition, agent, kit, system, or the like for encapsulating a cell in each gel capsule.

In the present disclosure, information for each nucleic acid derived from each individual cell can be obtained by any method, but it can be preferable to obtain an amplified nucleic acid derived from each individual cell by a method comprising a step of individually encapsulating each of cells in a liquid droplet; a step of gelating the liquid droplets to produce gel capsules; a step of immersing the gel capsules in one or more lysis reagents to lyse the cells; and a step of contacting the polynucleotide with an amplification reagent to amplify the polynucleotide in the gel capsules by using a sample comprising a microbiota in order to conveniently obtain information for each nucleic acid derived from each individual cell from a large number of cells. In one embodiment of the present disclosure, the step of contacting the polynucleotide with an amplification reagent to amplify the polynucleotide in the gel capsules can amplify the polynucleotide while maintaining a gel-like state within a gel capsule.

In one embodiment, liquid droplets can be prepared by allowing a cell to flow in a microchannel and shearing a suspension with oil to encapsulate the cell. In some embodiments, a gel capsule can be a hydrogel capsule.

Materials of a gel capsule can comprise agarose, acrylamide, photocurable resin (e.g., PEG-DA), PEG, gelatin, sodium alginate, Matrigel^{™}, collagen, or the like. A liquid droplet can be gelated by configuring the liquid droplet to comprise a material of a gel capsule and cooling the prepared liquid droplet. Alternatively, gel can be formed by stimulating a liquid droplet by light or the like. For example, a material of a gel capsule can be included in a suspension of cells or cell-like constructs so that the material of a gel capsule is included in a liquid droplet.

A gel capsule can be a hydrogel capsule. As used herein, "hydrogel" refers to gel in which a solvent or dispersion medium retained by a polymeric substance or colloidal particle mesh structure is water.

The method of the present disclosure can comprise a step of amplifying a nucleic acid derived from each individual cell in the population of cells. Thus, the system, kit, composition, or the like of the present disclosure can comprise a nucleic acid amplifying agent for amplifying a nucleic acid. The amplification step can comprise a step of immersing the gel capsules in one or more lysis reagents to lyse the cells, and a genomic DNA of the cells or a polynucleotide comprising said portion elutes out into the gel capsules and is retained in the gel capsules.

At least one lysis reagent can be selected from the group consisting of lysozyme, labiase, Yatalase, achromopeptidase, protease, nuclease, zymolyase, chitinase, lysostaphin, mutanolysin, sodium dodecyl sulfate, sodium lauryl sulfate, potassium hydroxide, sodium hydroxide, phenol, chloroform, guanidine hydrochloride, urea, 2-mercaptoethanol, dithiothreitol, TCEP-HCl, sodium cholate, sodium deoxycholate, Triton X-100, Triton X-114, NP-40, Brij-35, Brij-58, Tween 20, Tween 80, octyl glucoside, octyl thioglucoside, CHAPS, CHAPSO, dodecyl-p-D-maltoside, Nonidet P-40, and Zwittergent 3-12.

It is desirable to use a lysis reagent or a combination of lysis reagents that is potent to a certain extent when amplifying or analyzing a nucleic acid for each single cell for various microbes.

Optionally, a step of removing a contaminant from gel capsules can be included after lysing cells. Since a contaminant that remains within a gel capsule after lysing inhibits subsequent polynucleotide amplification, it can be desirable to discharge the inhibiting substance out of the gel capsule by passing a suitable detergent solution through the gel capsule. To complete such operations within a gel capsule, it can be desirable to have a hydrogel structure, which achieves permeation and discharge of various drug solutions and cell lysate while retaining a polynucleotide within the gel capsule. A residual reagent can be diluted while retaining a genetic material by using a gel capsule. This step can also be repeated. Downstream operations such as an amplification reaction can be performed smoothly by diluting a reagent to a level where inhibition is not manifested.

Examples of contaminants include components that are not derived from a cell remaining in a sample, components of lysed cells (e.g., cell membrane, intracellular protein, and polysaccharide), nucleic acid binding proteins (e.g., histone), lysis reagents, and excess labeling agents. Contaminants can be removed through immersion into a buffer, centrifugation, filtration, or the like while retaining a nucleic acid that is the subject of analysis in a gel capsule by using a gel capsule. If a labeling agent is to be added after gel capsule encapsulation, it is preferable to include a washing step to remove free labeling agents (e.g., EMA). During the washing step, a labeling agent bound to a nucleic acid can be maintained in a bound state while removing a protein bound to a nucleic acid which obstructs analysis.

Furthermore, an amplification step can comprise a step of contacting the polynucleotide with an amplification reagent to amplify the polynucleotide in the gel capsules. In this amplification step, information on whether a cell is alive or dead can be obtained with a labeling agent described above or the like. Since a reaction that involves heating (80 degrees or higher) can result in re-dissolving of gel (e.g., agarose gel), the individual particular shape can be disrupted to render single cell isolation ineffective. In such a case, an enzymatic reaction at about 60 degrees or lower is desirable in order to maintain the gel liquid droplet shape. Multiple displacement amplification can be performed within such a temperature range and is preferable in that every region of genomic DNA can be amplified. Examples of enzymes that are used include phi29 polymerase, Bst polymerase, Aac polymerase, and recombinase polymerase. In such a case, a step of contacting a polynucleotide with an amplification reagent to amplify the polynucleotide in gel capsules can amplify the polynucleotide while maintaining a gel state in the gel capsules.

When performing PCR in order to detect a specific cell (e.g., specific microbe), a specific primer is generally used in accordance with the microbe. However, it is preferable to use a random primer when performing amplification of the whole genome. There are thousands to 10s of thousands of types of mRNA in a cell based on genomic DNA, with a high molecular weight individually. For this reason, expression analysis on RNA is intended to find the absolute (relative) gene type or expression level. Thus, which gene is expressed at which level can be quantified by reading only a portion of a gene (10s of bases). If a genomic DNA is targeted, a genomic DNA only has one molecule in one cell in principle, so that it may be necessary to increase sequence information of the only molecule without any omission in order to decode all of the millions of bases thereof. Processing in a gel capsule is advantageous for such amplification. It is also advantageous for single cell analysis to obtain a nucleic acid for sequencing as a whole instead of fragmented information from portions of one cell.

### (Analysis)

Cells that can be targeted in analysis of a cell population of the present disclosure are any number that is two or greater, which can be, for example, 10 or more, 50 or more, 100 or more, 500 or more, 1000 or more, 5000 or more, 10000 or more, 50000 or more, 100000 or more, 500000 or more, 1 million or more, 5 million or more, or 10 million or more. Analysis of a cell population of the present disclosure can use information on each nucleic acid derived from each individual cell from a greater number of cells than using a conventional single cell reaction system such as 0.2 mL or 1.5 mL microtube reaction system.

Analysis of composition of a population of cells in the present disclosure can comprise identifying an absolute number of various cells in the population of cells. Since the present disclosure can obtain information on whether a cell is alive or dead for each individual cell, analysis of composition of a population of cells can comprise identifying an absolute number of various cells in live cells in the population of cells. Genomic sequence data of each individual cell can be obtained from a sample comprising an amplified nucleic acid derived from each individual cell in a population of cells, which can be performed live cell specifically.

The present disclosure can detect and screen separate individuals specifically by referring to the structure or sequence of nucleic acids prepared in parallel from various cells or other biomolecules prior to analysis on the sequence information of individual cells as a whole (e.g., genomic sequence decoding). Specifically, a method can comprise a step of selecting a sample comprising an amplified nucleic acid to be analyzed from samples comprising an amplified nucleic acid derived from each individual cell. Analysis of an amplified nucleic acid can comprise identifying a type of each individual cell. Selection can be performed by utilizing information on whether a cell is alive or dead.

In one embodiment, a sample can be selected based on the presence/absence of a specific genetic sequence, yield of a specific gene, or total nucleic acid yield. In some embodiments, a sample can be selected in the presence of a specific genetic sequence or in the absence of a specific genetic sequence. In some embodiments, a sample can be selected when the yield of a specific gene is greater than a baseline yield or lower than a baseline yield. In some embodiments, a sample can be selected when the total nucleic acid yield is greater than a baseline yield or lower than a baseline yield.

In a specific embodiment, the presence/absence of a specific genetic sequence is detected by means selected from the group consisting of a reagent that specifically detects a specific genetic sequence, agarose gel electrophoresis, microchip electrophoresis, PCR, qPCR, and gene sequencing (Sanger sequencing or NGS). Examples of reagents that specifically detect a specific genetic sequence include antibodies, probes, DNA binding fluorescent dyes, and fluorescent dye binding nucleotides in some embodiments.

In a specific embodiment, the yield of a specific gene or total nucleic acid yield can be measured through measuring absorbance, measuring fluorescence, agarose gel electrophoresis, or microchip electrophoresis. A method can comprise a step of detecting a nucleic acid having a specific sequence in samples comprising an amplified nucleic acid from each individual cell. The step of detecting a nucleic acid having a specific sequence can comprise amplifying a nucleic acid having a specific sequence and sequencing the nucleic acid.

The method of the present disclosure can comprise a step of obtaining genomic sequence data of each individual cell from a sample comprising an amplified nucleic acid derived from each individual cell in the population of cells. By obtaining genomic sequence data, not only information as just a sequence, but information from the viewpoint of the function served by a sequence can be obtained for individual cells in a cell population.

The system, kit, composition, or the like of the present disclosure can comprise means for obtaining data of a nucleic acid sequence. Such means can be any means, (e.g., kit, agent, or the like) as long as genomic sequence data of each individual cell can be obtained from a sample comprising an amplified nucleic acid derived from each individual cell in a cell population. Such means can provide a function of obtaining not only information as just a sequence, but also information from the viewpoint of the function served by a sequence for individual cells in a cell population after obtaining genomic sequence data.

Genomic sequence data to be analyzed can be selected from genomic sequence data for each individual cell. The amount of information is high for genomic sequence data. Limitation of the amount to be processed leads to reduction in labor and time.

Selection can comprise evaluating the presence/absence of a specific genetic sequence and/or identity with a specific genetic sequence. In some embodiments, identity with a specific genetic sequence can be evaluated by using BLAST or the like. In a specific embodiment, selection can screen nucleic acid information by cells based on the presence/absence of a specific genetic sequence. In another embodiment, selection can screen nucleic acid information by cells based on identity of a specific genetic sequence with nucleic acid information derived from two or more cells. In some embodiments, selection can be based on having identity of a certain level or higher or identity of a certain level or lower. In a specific embodiment, identity can be 50% or higher 55% or higher, 60% or higher, 65% or higher, 70% or higher, 75% or higher, 80% or higher, 85% or higher, 90% or higher, 91% or higher, 92% or higher, 93% or higher, 94% or higher, 95% or higher, 96% or higher, 97% or higher, 98% or higher, 99% or higher, or 100%. In another embodiment, identity can be 50% or lower, 45% or lower, 40% or lower, 35% or lower, 30% or lower, 25% or lower, 20% or lower, 15% or lower, 10% or lower, 9% or lower, 8% or lower, 7% or lower, 6% or lower, 5% or lower, 4% or lower, 3% or lower, 2% or lower, 1% or lower, or 0%.

### (Cell population)

In the present disclosure, a population of cells can be a population of animal somatic cells, plant somatic cells, or germ cells, a population of microbial cells, or a combination thereof. The cells or cell-like constructs of the present disclosure are not particularly limited. Examples thereof include microbes (e.g., bacteria, fungi, and unicellular animals) and cells of a multicellular organism (e.g., somatic cells, germ cells, cultured cells, tumor cells, animal cells, and plant cells). In the present disclosure, a population of cells can be a microbiota or a microbial formulation.

Examples of microbes that can be targeted in the present disclosure include, but are not limited to, eubacteria, E. coli, Bacillus subtilis, cyanobacteria, cocci, Bacillus, Spirillum, gram negative bacteria, gram positive bacteria, archaea, fungi, individual cells of a multicellular organism, and any combination thereof. Examples of microbiota include bacterial flora. Since a microbiota can comprise a plurality of microbes with different cellular properties (e.g., presence/absence of a cell wall, etc.), it can be preferable in the analysis of the present disclosure to employ means capable of obtaining nucleic acid information for each single cell or an amplified nucleic acid for each single cell in the same manner regardless of the microbe species.

A microbiota can be, but is not limited to, gut microbiota, skin microbiota, oral microbiota, nasal cavity microbiota, vaginal microbiota, soil microbiota, rhizosphere microbiota, river/sea water microbiota, activated sludge microbiota, insect symbiotic microbiota, animal symbiotic microbiota, or the like. Gut bacterial flora is extensively studied for its effect on human health and is one of the preferred microbiotas as the subject of analysis of the present disclosure.

In the present disclosure, information from analysis of nucleic acid sequence information that is not derived from each individual cell but is derived from the same microbiota can be further combined for analysis. Examples of analysis include metagenomics. Metagenomics is a method of extracting and collecting nucleic acid, gene, or DNA of a microbe in an environment as a whole without a process of culturing to comprehensively study structure thereof (base sequences). Although it is unknown which microbe an individual nucleic acid or gene is derived from, information on a group of genes of a community of microbes in an environment can be obtained. Such a method is known as metagenomics.

Evaluation of microbiota composition can include identifying an absolute number of various microbes in a microbiota. An absolute number of various microbes can be identified by identifying the microbe species for each amplified nucleic acid derived from each individual cell. The microbe species can be identified, for example, by identifying the presence/absence of a specific genetic sequence.

If genomic sequence data is available, microbiota composition can be evaluated by comparing long genetic sequences in each microbe. Long sequences can be, for example, genetic sequences extracted from de novo assembly data (e.g., a set of 16S genetic sequences, common genes, etc.). Comparison of such long genetic sequences can not only enhance the accuracy of evaluation, but also enable evaluation of a function in a microbiota. For example, with only sequence information, the function of the entire microbiota cannot be understood without separate information for each function, even if the presence of a plurality of species is known. It is possible to obtain information on the quantity of species with a possibility of having a specific activity (e.g., enzymatic activity) in a microbiota or the like based on genetic information.

### (Application)

The present disclosure can be used, for example, in evaluating quality of an FMT (fecal microbiota transplant) specimen. Evaluation such as bacteria microbiota analysis or live bacteria count evaluation can be necessary for stable supply or quality assurance of an FMT specimen. For FMT specimens, bacteria microbiota varies by each specimen, with different bacteria subjected to study and bacteria count. Since the technology of the present disclosure can be applied even if the target species' component ratio or sequence information are unknown, the technology is understood to be technically superior over conventional methods of culturing specific bacteria or observation methods.

The present disclosure can be used, for example, in evaluating quality of a microbial formulation. Evaluation such as bacteria microbiota analysis or live bacteria count evaluation can be necessary in the manufacture quality assurance, stable supply, or quality assurance upon administration for a microbial formulation. A microbial formulation is prepared as a cocktail of a plurality of species of bacteria. If it is necessary to distinguish related species of bacteria, DNA sequence information is required because microscope observation is not capable thereof. Further, an expected property may not be obtained due to mutation accumulation associated with long-term culture or manufacture or the like. It is understood that the ability to perform genome level analysis of diverse bacterial species with the technology of the present disclosure can be utilized effectively in terms of lot management between formulations.

The present disclosure can be used, for example, in inspecting food microbes. Sanitary indicative bacteria (contamination indicative bacteria) for evaluating sanitary quality of food products include common bacteria count (live bacteria count). However, official analytical methods that require culturing are time consuming and can be used only for specific bacteria. It is understood that the technology of the present disclosure can be advantageously applied if highly accurate analysis is required.

The present disclosure can be used, for example, in investigating live bacteria in an environment. Environmental contamination due to pathogenic bacteria in an environment can be studied. Examples of pathogenic bacteria include Legionella, 0-157, multidrug resistant bacteria, and the like. The official analytical methods that require culturing are time consuming and can be used only for specific bacteria. It is understood that the technology of the present disclosure can be advantageously applied if highly accurate analysis is required. For example, multidrug resistant bacteria that are present as live bacteria in an environment can be identified, bacteria count can be evaluated, or the like. Active bacteria with activity in various environments such as the gut, soil, or rhizosphere can be identified. The technology of the present disclosure is effective for preferentially obtaining genomic sequence information by specifically detecting live bacteria understood to contribute to a host in particular in excellent soil, disease resistant rhizosphere, or the like.

The method of the present disclosure can be used, for example, in evaluating quality of microbe derived animal feed, fermented food products, or microbe containing supplement, profiling of live bacteria in activated sludge, evaluating the presence of live microbes in an environment, evaluating the effect or spectrum of an antibacterial drug, evaluating a sterilization method, profiling of live bacteria in a biofilm, or the like.

### (System)

In another aspect of the present disclosure, a system for analyzing biome composition of a cell population can be provided. A system can comprise means for implementing a method comprising any feature described in another item herein or a step thereof.

A system can comprise an apparatus for amplifying a polynucleotide in a cell or the like. In particular, an apparatus or the like can be capable of amplifying a polynucleotide in a cell at a single cell level. An apparatus can comprise a liquid droplet preparation unit for encapsulating cells in each liquid droplet in a unit of one cell or construct; a gel capsule producing unit for gelating the liquid droplets to produce gel capsules; a labeling unit for adding a labeling agent and labeling cells or gel capsules comprising a cell; a lysis reagent immersing unit for immersing gel capsules in a lysis reagent; a removing unit for removing a contaminant from the gel capsules; and/or an amplification reagent immersing unit for immersing the gel capsules in an amplification reagent. A system or apparatus can further comprise a screening unit for screening gel capsules and housing the gel capsules in a container. A system or apparatus can comprise a detecting unit for detecting a label to obtain information on whether a cell is alive or dead.

A system or apparatus can optionally comprise a medium for encapsulating a cell, gel capsule material, labeling agent, nucleic acid degradation promoting agent, lysis reagent, amplification reagent, nucleic acid binding fluorescent substance capable of detecting amplification of a nucleic acid, a reagent used in sequencing of a nucleic acid (e.g., polymerase, primer set (may also comprise a barcode sequence), or the like) or other reagents. A reagent that is known in the art in addition to those described elsewhere herein can be used as the reagent.

An apparatus or system can further comprise a sequencing unit for sequencing a nucleic acid sequence in a polynucleotide amplified in an amplification reagent immersing unit. A sequencing unit can be provided integral with the apparatus described above or provided as a separate apparatus in a system. A sequencing unit can be an instrument for performing a Sanger method, Maxam-Gilbert method, single-molecule real-time sequencing (e.g., Pacific Biosciences, Menlo Park, California), ion semiconductor sequencing (e.g., Ion Torrent, South San Francisco, California), sequencing by synthesis, pyrosequencing (e.g., 454, Branford, Connecticut), sequencing by ligation (e.g., SOLiD sequencing of Life Technologies, Carlsbad, California), sequencing by synthesis and a reversible terminator (e.g., Illumina, San Diego, California), nucleic acid imaging technology such as a transmission electron microscopy, nanopore sequencing, or the like.

A system or apparatus can comprise means for detecting/measuring an amplified gene. For example, a flow cytometer which is suitable for handling the shape of a gel capsule can be provided integral with the apparatus described above, or provided as a separate apparatus in the system. Means for detecting/measuring an amplified gene can comprise means for performing a detection reaction (e.g., thermal cycler and a suitable reagent) and/or means for detecting a signal (light sensor, camera, and suitable means for analysis). Means for detecting/measuring an amplified gene can also function as a detecting unit for detecting a label to obtain information on whether a cell is alive or dead.

A system or apparatus can comprise a computing unit that can be configured to perform any information processing described elsewhere herein. A computing unit can be provided integral with the apparatus described above, or as a separate apparatus (computer) in the system. In another aspect of the present disclosure, a computing unit can be provided with a program and a storage medium with the program recorded thereon for implementing the method of the present disclosure for information processing described elsewhere herein. A computing unit can optionally comprise such a program and/or a storage medium with the program recorded thereon.

### (Kit)

One aspect of the present disclosure provides a kit that can be used in the method of the present disclosure. The present disclosure can provide a kit for analyzing composition of a cell population. A kit can comprise a material of a gel capsule. Use of a gel capsule is advantageous for amplifying a nucleic acid in a cell or a cell-like construct at a single cell level as described elsewhere herein. A gel capsule can be used for analysis of microbe composition as described herein. A kit can comprise, for example, a material of a gel capsule and optionally one or more reagents. A reagent known in the art, in addition to those described elsewhere herein, can be used as the reagent. A kit can comprise a labeling agent, nucleic acid degradation promoting agent, or nucleic acid binding fluorescent substance capable of detection amplification of a nucleic acid that is described herein or is known in the art.

A kit for analyzing microbiota composition can comprise a lysis reagent. The lysis reagent can comprise at least one selected from the group consisting of lysozyme, labiase, Yatalase, achromopeptidase, protease, nuclease, zymolyase, chitinase, lysostaphin, mutanolysin, sodium dodecyl sulfate, sodium lauryl sulfate, potassium hydroxide, sodium hydroxide, phenol, chloroform, guanidine hydrochloride, urea, 2-mercaptoethanol, dithiothreitol, TCEP-HCl, sodium cholate, sodium deoxycholate, Triton X-100, Triton X-114, NP-40, Brij-35, Brij-58, Tween 20, Tween 80, octyl glucoside, octyl thioglucoside, CHAPS, CHAPSO, dodecyl-p-D-maltoside, Nonidet P-40, and Zwittergent 3-12. A lysis reagent is useful for obtaining an amplified polynucleotide, especially an amplicon throughout the entire genome at a single cell level.

A kit can comprise a nucleic acid amplification reagent. Examples of amplification reagent include polymerase, primer set (may include a barcode sequence), base mix, suitable buffer, and the like. A kit can comprise a reagent such as a reagent used in sequencing of a nucleic acid (e.g., polymerase, primer set (may include a barcode sequence), or the like). For example, a reagent for amplifying or sequencing a specific gene through Sanger sequencing or NGS (polymerase, primer set (may include a barcode sequence), etc.) can be included. A kit can also comprise a reagent that can be used in detection or measurement of a specific sequence such as a nucleic acid binding dye or fluorescent label probe. The presence/absence of a specific sequence can be measured with an instrument for detecting or measuring an amplified gene (flow cytometer or the like) by using these reagents.

A kit can comprise means for collecting a sample. A kit can also comprise means for preserving the collected sample. Examples of means for collecting a sample include a syringe, swab, biopsy punch, urine sample container, stool sample container, saliva sample container, medical tape, and the like. Examples of means for preservation include a coolant (e.g., refrigerant, dry ice, and liquid nitrogen), preservation solution (e.g., solutions containing guanidine hydrochloride, ammonium sulfate, ethanol, or the like), and the like.

One aspect of the present disclosure can provide a kit comprising a specimen container comprising a preservation solution for analyzing composition of a cell population. A preservation solution can comprise, for example, a guanidine solution (e.g., FS-0007 or FS-0008, TechnoSuruga Laboratory) or ethanol (e.g., OMR-200 or OM-501, DNA genotek). An effect such as an excellent genome decoding rate (completion rate) or identification of many organism lines in a sample can be attained in subsequent single cell level analysis even after storage at normal temperature for a certain period at a hospital facility or the like by using a kit comprising such a preservation solution. Further, convenience is significantly higher compared to a stool storage method that is conventionally materialized as a frozen stool.

A preservation solution is generally used to enhance the stability of a nucleic acid. For example, a guanidine solution, alcohol, and the like are used to inhibit the action of an enzyme, which cause protein denaturation and advances decomposition of a nucleic acid. It can be preferably to main morphological stability as a cell during preservation of a sample in the method of the present disclosure. This is because cell disruption makes encapsulation into a droplet and data acquisition difficult due to many cellular debris, or the like. For this reason, it was expected that a preservation solution that causes protein denaturation would be disadvantageous for the stability of cellular morphology, but it was found in the Examples herein that many cells unexpectedly retain excellent morphology after preservation in a preservation solution, and the preservation solution is particularly suitable in the method of the present disclosure.

### (Preferred Embodiments)

Preferred embodiments of the present disclosure are described below. Embodiments described below are provided to facilitate the understanding of the present disclosure. It is understood that the scope of the present disclosure should not be limited to the following descriptions. Thus, it is apparent that those skilled in the art can make appropriate modifications within the scope of the present disclosure by referring to the descriptions herein. It is also understood that the following embodiments can be used independently or as a combination thereof.

A reagent comprising a selective membrane permeable dye (e.g., EMA) is added to a sample. EMA and a nucleic acid are then attached by light irradiation. EMA permeates inside of dead bacteria with a damaged membrane and selectively modifies a DNA. While unstained DNA derived from a live cell is amplified by PCR, a DNA derived from a dead cell with an EMA attached thereto reaches a state where the DNA cannot be amplified. A sample is prepared by excluding treatment solution comprising EMA from the sample, and a gel capsule comprising a polynucleotide derived from a single cell genome that has been amplified and retained is prepared by the method described herein. In this regard, the polynucleotide is selectively amplified and prepared in a gel capsule comprising live bacteria. Such gel capsules are stained with a DNA binding fluorescent dye or the like, and fluorescence positive capsules are counted by flow cytometry. The number of fluorescence positive capsules in a certain volume corresponds to the total introduced live bacteria count.

Next, gel capsules are separated and collected in a microplate for each single cell by a method described herein. Furthermore, a whole genome amplification reaction is performed using a polynucleotide in each gel capsule as a template in the plate. This is used as the library master plate. A portion of the reaction solution in the library master plate is then fractionated to prepare a replica plate. Amplification is performed using the replica as a template with a target gene specific primer set. Subsequently, the sequence of the amplicon is identified by Sanger sequencing or the like to identity the microbe species of each sample.

More effectively, a barcode sequence is included in the primer set described above to perform an amplification reaction that gives a different barcode to each sample. PCR products derived from a plurality of replica plates are pooled to perform next generation sequencing. With a barcode sequence, the plate/well number is then identified, and the sequence of a PCR product is identified to identify microbe species of hundreds to thousands of samples at once. The primer set described above can also be a mixture of primer sets targeting a plurality of gene regions. More specifically, the primer set is selected from primer sets targeting the v3-v4, v1-v2 region, or the like of a 16S rRNA gene, primer set that distinguishes 18S rRNA, ITS, other bacteria, archaea, fungi, or the like, etc. If a plurality of types of the primer sets described above are simultaneously used, bacteria, archaea, and fungi can be simultaneously detected.

When it is difficult to design a primer set of a target gene sequence or in order to determine a target gene region with high accuracy, a method of performing whole genome sequencing and searching for a target gene region from digital sequence information is effective. Alternatively, the objective can be to determine an unknown live bacteria genome by performing whole genome sequencing. Whole genome sequencing is performed from a sample of a replica plate, and a genetic sequence is extracted from digital sequence information obtained from next generation sequencing to obtain data that identifies bacteria species or gene. For the gene to be search or extracted, a candidate would be a group of genes associated with a specific enzyme or secondary metabolite production from a gene region that distinguishes bacteria, archaea, fungi, or the like, such as 16S rRNA gene, 18S rRNA gene, ITS, other.

The number of live cells of each microbe species in a sample can be counted by identifying and counting microbe species contained in each gel capsule fractionated into a well plate or the like by one of the methods described above, together with the total live bacteria count measured initially. In this reaction, every PCR reaction is performed separately, and microbe species are identified and counted for each barcode. Thus, digital count data reflecting the cell count is obtained. Specifically, data that avoids a bias due to the presence/absence of known sequence information, culturable/not culturable, or the number of copies of a gene, which was a problem in the past, is obtained. Further, rare cells which are present at a relative ratio of 1% or less can be compared with substantial total number.

One embodiment of the method of the present disclosure includes a method comprising: a step of adding EMA to a population of cells and irradiating light; a step of individually encapsulating each of cells of the population of cells in liquid droplets; a step of gelating the liquid drops to produce gel capsules; a step of immersing the gel capsules in one or more lysis reagents to lyse the cells, wherein a genomic DNA of the cells or a polynucleotide comprising said portion elutes out into the gel capsules; a step of removing a contaminant from the gel capsules; a step of contacting the polynucleotide with an amplification reagent to amplify the polynucleotide in the gel capsules, wherein a polynucleotide bound to EMA is not amplified; a step of detecting an amplified polynucleotide; a step of selecting gel capsules comprising a sample derived from a live bacterium; a step of counting gel capsules comprising a sample derived from a live bacterium; and a step of analyzing a polynucleotide amplified in a gel capsule comprising a sample derived from a live bacterium. Another embodiment of the method of the present disclosure includes a method comprising: a step of individually encapsulating each of cells of the population of cells in a liquid droplet; a step of gelating the liquid droplets to produce gel capsules; a step of adding EMA to the gel capsules and irradiating light; a step of washing the gel capsules; a step of immersing the gel capsules in one or more lysis reagents to lyse the cells, wherein a genomic DNA of the cells or a polynucleotide comprising said portion elutes out into the gel capsules; a step of removing a contaminant from the gel capsules; a step of contacting the polynucleotide with an amplification reagent to amplify the polynucleotide in the gel capsules, wherein a polynucleotide bound to EMA is not amplified; a step of detecting an amplified polynucleotide; a step of selecting gel capsules comprising a sample derived from a live bacterium; a step of counting gel capsules comprising a sample derived from a live bacterium; and a step of analyzing a polynucleotide amplified in a gel capsule comprising a sample derived from a live bacterium. Only a portion of the steps in these methods can be used as appropriate, as long as the objective of the present disclosure is achieved. Those skilled in the art understand that a step can be replaced with an equivalent technology that is described herein or is known in the art, or such a technology can be added and used.

### (Other embodiments)

The present disclosure can provide a system for analyzing composition of a population of cells. The system can comprise means for obtaining information on whether a cell is alive or dead for each individual cell in the population of cells; and/or means for analyzing an amplified nucleic acid derived from each individual cell in the population of cells. An embodiment of the present disclosure can provide a system or kit for analyzing composition of a population of cells. The system or kit can comprise a labeling agent for differentially labeling a live cell and a dead cell; means for encapsulating the cells in gel capsules; means for amplifying a nucleic acid; and/or means for analyzing the nucleic acid. The present disclosure can provide a composition comprising a labeling agent for differentially labeling a live cell and a dead cell. Said composition can be for use in a method of analyzing composition of a population of cells. The method can comprise: a step of obtaining information on whether a cell is alive or dead for each individual cell in the population of cells; and a step of analyzing an amplified nucleic acid derived from each individual cell in the population of cells. The present disclosure can also provide a composition for use in a method of individually encapsulating each of cells of a population of cells in a gel capsule for analysis, comprising a labeling agent for differentially labeling a live cell and a dead cell.

The present disclosure provides a system or kit. The system or kit can comprise: a reagent for distinguishing whether a cell is alive or dead (ethidium monoazide (EMA) or propidium monoazide (PMA)); light irradiation means; means for individually encapsulating each of cells in a liquid droplet; means for gelating the liquid droplets to produce gel capsules; a lysis reagent for the gel capsules, wherein the lysis reagent is added and used so that a genomic DNA of the cells or a polynucleotide comprising said portion elutes out into the gel capsules; means for removing a contaminant from the gel capsules; an amplification reagent for a polynucleotide, wherein the amplification reagent is used so that the polynucleotide is contacted with the amplification reagent to amplify the polynucleotide in the gel capsules, but a polynucleotide bound to EMA is not amplified; means for detecting a polynucleotide; means for selecting gel capsules comprising a sample derived from a live bacterium; means for counting gel capsules comprising a sample derived from a live bacterium; and/or means for analyzing a polynucleotide amplified in a gel capsule comprising a sample derived from a live bacterium.

The present disclosure can provide a composition for use in a method of analyzing composition of a population of cells, comprising a labeling agent for differentially labeling a live cell and a dead cell (e.g., ethidium monoazide (EMA) or propidium monoazide (PMA)), wherein the method comprises a step of obtaining information on whether a cell is alive or dead for each individual cell in the population of cells; and a step of analyzing an amplified nucleic acid derived from each individual cell in the population of cells.

The system for analysis of the present disclosure comprises a sample providing unit for providing a sample comprising an amplified nucleic acid derived from each individual cell in a sample and a composition evaluating unit for evaluating composition of a sample from a sample comprising an amplified nucleic acid derived from each individual cell in the sample. In this regard, the sample providing unit comprises: a liquid droplet encapsulating unit for individually encapsulating each of cells in a liquid droplet using a sample; a gel capsule producing unit for gelating the liquid droplet to produce gel capsules; a cell lysing unit, in which one or more lysis reagents for lysing cells are stored, for immersing the gel capsules in the one or more lysis reagents to lyse the cells, wherein the cell lysing unit is configured so that a genomic DNA of the cells or a polynucleotide comprising said portion elutes out into the gel capsules and is retained in the gel capsules in a state where the genomic DNA or a substance bound to said portion is removed; and a polynucleotide amplification reagent for amplifying the polynucleotide in a gel capsule.

Reference literatures such as scientific literatures, patents, and patent applications cited herein are incorporated herein by reference to the same extent that the entirety of each document is specifically described.

The present disclosure has been described while showing preferred embodiments to facilitate understanding. While the present disclosure is described hereinafter based on the Examples, the above descriptions and the following Examples are provided for the sole purpose of exemplification, not limitation of the present disclosure. Thus, the scope of the present disclosure is not limited to the embodiments and Examples that are specifically described herein and is limited only by the scope of claims.

### [Examples]

The Examples of the present disclosure are described hereinafter. For reagents, the specific products described in the Examples were used. However, the reagents can be substituted with an equivalent product from another manufacturer (Sigma-Aldrich, Wako Pure Chemical, Nacalai Tesque, R & D Systems, USCN Life Science Inc., or the like).

### (Example 1: Experimental example)

An E. coli K12 strain culture is prepared, and dead bacteria model samples are prepared by heating (95°C, 5 min) or alcohol treatment (suspended in 70% ethanol). Each of untreated (live bacteria) samples, dead bacteria model samples, and live/dead bacteria mixture samples is prepared. A bacterial pellet is suspended in 500 µL of phosphate buffered saline (PBS), treated using TakaraBio's Viable Bacteria Selection Kit for PCR in accordance with the protocol, and treated by addition of EMA, permeation, and light irradiation. The suspension is then centrifuged for 3 minutes at 15000 × g to harvest the bacteria. After centrifuging and washing bacterial pellets twice with PBS, the pellets are suspended in PBS to obtain an E. coli suspension. The cell concentration in the prepared cell suspension is measured, and an ultralow melting point agarose is added so that the final concentration would be 1.5% to prepare an E. coli suspension used in the preparation of gel capsules (final cell concentration: 1.5 × 10³ cells/µL).

In the following manner, E. coli is encapsulated into a microdroplet, and microdroplet gelation and whole genome amplification in a gel capsule are performed.

Microdroplets are prepared and E. coli is encapsulated into the microdroplets by using a microchannel manufactured by the inventors using polydimethylsiloxane (Sylgard 184: Dow Corning). While this Example uses a microchannel consisting of a first channel, second channel, third channel, and fourth channel wherein adjacent channels are arranged perpendicularly, a microchannel with an approximately T shape connection can also be used. The microchannel that is used in this Example has a width of 34 µm and a height of 50 µm, but the size of microchannel can be appropriately changed depending on the size of microdroplets to be prepared and the size of a cell to be encapsulated.

Next, E. coli suspension is introduced from the first channel (aqueous phase inlet) . Pico-Surf 1 (2% in Novec 7500) (Sphere Fluidics) (hereinafter, referred to as "oil") is introduced from the second channel and fourth channel (oil phase inlets) to shear the gut microbe suspension to prepare microdroplets with a diameter of 50 µm. The microdroplets are allowed to flow in the third channel 7 and collected in a 0.2 mL volume tube. About 450000 microdroplets are prepared at a rate of 500 droplets/second. The cell concentration within the microdroplets is 0.1 cells/droplet.

In this Example, the diameter of microdroplets is uniformly 50 µm to facilitate encapsulation of each individual cell into a microdroplet. In view of the size of a single cell, the diameter of a microdroplet is, for example, 1 to 250 µm and preferably 20 to 200 µm. The diameter of liquid droplets can be about 1 to 250 µm and more preferably about 10 to 200 µm such as about 1 µm, about 5 µm, about 10 µm, about 15 µm, about 20 µm, about 25 µm, about 30 µm, about 40 µm, about 50 µm, about 80 µm, about 100 µm, about 150 µm, about 200 µm, or about 250 µm.

While a plurality of microdroplets and oil are housed within a tube, the microdroplets accumulate on the top layer due to a lower specific gravity than oil.

Next, the tube is cooled for 15 minutes on ice. The microdroplets are gelated with an ultralow melting point agarose. The gelated microdroplets are used as gel capsules. Since the diameter of microdroplets is 50 µm, the diameter of gel capsules would also be 50 µm. The diameter of a gel capsule can be about 1 to 250 µm and more preferably about 10 to 200 µm such as about 1 µm, about 5 µm, about 10 µm, about 15 µm, about 20 µm, about 25 µm, about 30 µm, about 40 µm, about 50 µm, about 80 µm, about 100 µm, about 150 µm, about 200 µm, or about 250 µm. The diameter of a gel capsule can be the same as the liquid droplets to be prepared, but the diameter can change upon gelation. The diameter of a gel capsule is preferably 1 to 250 µm. The rate of permeation of the bacteriolytic reagents described below into each gel capsule can be more uniform if the diameters of gel capsules are uniform.

Next, 20 µL of 1H, 1H, 2H, 2H-perfluoro-1-octanol (SIGMA-ALDRICH) is added to a tube. After removing oil in the bottom layer, acetone (Fujifilm Wako Pure Chemical) (500 µL) and isopropanol (Fujifilm Wako Pure Chemical) (500 µL) are added in order, centrifuged, and washed to remove oil. In this Example, contaminants include oil permeated into a gel capsule. Furthermore, 500 µL of PBS is added and the mixture is centrifuged and washed three times so that the gel capsules are suspended in an aqueous layer (PBS). The gel capsules accumulate on the bottom layer due to a higher specific gravity than an aqueous layer.

Subsequently, the gel capsules are sequentially immersed in a bacteriolytic reagent used as a lysis reagent. The portions other than the substance of interest to be collected such as cell walls are dissolved inside the gel capsules so that genomic DNA would elute out within the gel capsules.

Specifically, lysozyme (10 U/µL) (R1804M, Epicentre), which is a type of a bacteriolytic reagent, is added to a tube to lyse cells. Next, achromopeptidase (850 U/mL) (015-09951, Fujifilm Wako Pure Chemical), which is a type of a bacteriolytic reagent, is added to the tube. Next, protease K (1 mg/mL) (MC5005, Promega) and sodium dodecyl sulfate (SDS) 0.5% (71736-100ML, SIGMA-ALDRICH), which are types of bacteriolytic reagents, are added to the tube. Cells are lysed then centrifuged and washed 5 times. Components other than genomic DNA of lysed cells and protease (contaminant) are removed from the tube. Subsequently, the gel capsules are immersed in Buffer D2 (QIAGEN), which is an aqueous solution comprising potassium hydroxide that is a type of a bacteriolytic reagent, to dissolve the residual components and denature genomic DNA. The bacteriolytic reagents used in this Example are lysozyme, achromopeptidase, protease K, sodium dodecyl sulfate, and Buffer D2 as described above. Although potassium hydroxide is also used in a common DNA amplification reaction step, potassium hydroxide also has a bacteriolytic effect, so that potassium hydroxide is used as one of the bacteriolytic reagents in this Example. Since a gel capsule is immersed in a bacteriolytic reagent for a short period of time, eluted genomic DNA would not flow out of the gel capsule due to the bacteriolytic reagent and remains within the gel capsule. In this Example, contaminants include bacteriolytic reagents that have permeated into a gel capsule.

In this Example, cells are centrifuged and washed only before adding Buffer D2 after lysing the cells by sequentially adding lysozyme, achromopeptidase, and protease K and adding sodium dodecyl sulfate. This can result in a sufficient washing effect. However, cells can be centrifuged and washed after lysing the cells with each bacteriolytic reagent.

In this manner, genomic DNA of interest can be collected by lysing cells with a plurality of types of bacteriolytic reagents. By centrifuging and washing after immersion into a bacteriolytic reagent, contaminants such as components other than polynucleotide of lysed cells and bacteriolytic reagent can be removed to purify genomic DNA without obstructing the subsequent genomic DNA amplification reaction.

An amplification reagent is added to a tube comprising a gel capsule retaining genome DNA denatured in a potassium hydroxide solution (Buffer D2) to immerse the gel capsule in the amplification reagent. Specifically, MDA (Multiple Displacement Amplification) using a strand displacement DNA synthase, phi29 DNA polymerase, is utilized. In this regard, a 3 hour whole genome amplification reaction is performed by immersion in a whole genome amplification reaction reagent REPLI-g Single Cell Kit (QIAGEN) (S1000 thermal cycler, Bio-Rad). The amplification reagent (REPLI-g Single Cell Kit) contains a component that neutralizes the potassium hydroxide solution (Buffer D2).

The gel capsules are stained with a fluorescent DNA intercalater. Gel capsules exhibiting fluorescence are counted by microscopy and flow cytometer and are individually collected. As a result, a gel capsule exhibiting fluorescence is not observed from dead bacteria model samples. In a mixed sample, about half of the untreated (live bacteria) samples presents a fluorescent signal. This is the ratio corresponding to the mixture ratio of live bacteria/dead bacteria.

After dissolving gel capsules by heating individual collected gel capsules at 65°C, secondary amplification is performed by MDA within a well of each plate to prepare a library master plate comprising 10 µL of DNA amplicon for each well. 39 µL of nuclease free water is dispensed in each well of a new plate, and 1 µL of the DNA amplicon in the library master plate is added to prepare a 40-fold diluent of the library master plate. Next, the DNA concentration is quantified with a Qubit fluorometer (Thermo Fisher Scientific) by using 1 µL of the diluent. PCR is performed on the V3-V4 regions of 16S rRNA gene by using 1 µL of the diluent as a template. After checking the presence/absence of a PCR product from agarose gel electrophoresis, sequencing analysis is performed on a portion of the samples observed to have amplified by using the Sanger method. Homology search is performed using BLAST on the resulting sequencing information. The 16S rRNA sequence derived from E. coli as expected is studied from the prepared library master plate.

### (Example 2: Obtaining information on whether a cell is alive or dead and sequence for each gel capsule)

An E. coli K12 strain culture was prepared, and dead bacteria model samples were prepared by heating (95°C, 5 min). Bacterial pellets were suspended in 500 µL of phosphate buffered saline (PBS). Some were treated using TakaraBio's Viable Bacteria Selection Kit for PCR in accordance with the protocol, and treated by addition of EMA, permeation, and light irradiation. The suspension was then centrifuged for 3 minutes at 15000 × g to harvest the bacteria. After centrifuging and washing the bacterial pellets twice with PBS, the pellets were suspended in PBS to obtain an E. coli suspension. The cell concentration in the prepared cell suspension was measured, and an ultralow melting point agarose was added so that the final concentration would be 1.5% to prepare an E. coli suspension used in the preparation of gel capsules (final cell concentration: 1.5 × 10³ cells/µL). Some other dead bacteria model samples were used to prepare a suspension with the same concentration without EMA treatment.

E. coli was encapsulated into microdroplets as described below. Gelation of the microdroplets and whole genome amplification within gel capsules were performed. Microdroplets were prepared and E. coli was encapsulated into the microdroplets by using a microchannel manufactured by the inventors using polydimethylsiloxane (Sylgard 184: Dow Corning). This Example used a microchannel consisting of a first channel, second channel, third channel, and fourth channel wherein adjacent channels are arranged perpendicularly. The microchannel used in this Example had a width of 34 µm and a height of 50 µm.

Next, the E. coli suspension was introduced from the first channel (aqueous phase inlet) . Pico-Surf 1 (2% in Novec 7500) (Sphere Fluidics) (hereinafter, referred to as "oil") was introduced from the second channel and fourth channel (oil phase inlets) to shear the gut microbe suspension to prepare microdroplets with a diameter of 50 µm. The microdroplets were allowed to flow in the third channel 7 and collected in a 0.2 mL volume tube. About 450000 microdroplets were prepared at a rate of 500 droplets/second. The cell concentration within the microdroplets was 0.1 cells/droplet. In this Example, the diameter of the microdroplets was 50 µm. Next, the tube was cooled for 15 minutes on ice. The microdroplets were gelated with an ultralow melting point agarose. The gelated microdroplets were used as gel capsules.

Next, 20 µL of 1H, 1H, 2H, 2H-perfluoro-1-octanol (SIGMA-ALDRICH) was added to a tube. After removing oil in the bottom layer, acetone (Fujifilm Wako Pure Chemical) (500 µL) and isopropanol (Fujifilm Wako Pure Chemical) (500 µL) were added in order, centrifuged, and washed to remove oil. In this Example, contaminants include oil permeated into a gel capsule. Furthermore, 500 µL of PBS was added and the mixture was centrifuged and washed three times so that the gel capsules were suspended in an aqueous layer (PBS). The gel capsules accumulate on the bottom layer due to a higher specific gravity than teh aqueous layer.

Subsequently, the gel capsules were sequentially immersed in a bacteriolytic reagent used as a lysis reagent. The portions other than the substance of interest to be collected such as cell walls was lysed inside the gel capsules so that genomic DNA would elute out within the gel capsules. Specifically, lysozyme (10 U/µL) (R1804M, Epicentre), which is a type of a bacteriolytic reagent, was added to a tube to lyse cells. Next, achromopeptidase (850 U/mL) (015-09951, Fujifilm Wako Pure Chemical), which are types of bacteriolytic reagents, was added to the tube. Next, protease K (1 mg/mL) (MC5005, Promega) and sodium dodecyl sulfate (SDS) 0.5% (71736-100ML, SIGMA-ALDRICH), which are a type of a bacteriolytic reagent, were added to the tube. Cells were lysed then centrifuged and washed 5 times. Components other than genomic DNA of lysed cells and protease (contaminant) were removed from the tube. Subsequently, the gel capsules were immersed in Buffer D2 (QIAGEN), which is an aqueous solution comprising potassium hydroxide that is a type of a bacteriolytic reagent, to lyse the residual components and denature genomic DNA.

An amplification reagent was added to a tube comprising a gel capsule retaining genome DNA denatured in a potassium hydroxide solution (Buffer D2) to immerse the gel capsule in the amplification reagent. Specifically, MDA (Multiple Displacement Amplification) using a strand displacement DNA synthase, phi29 DNA polymerase, was used. In this regard, a 3 hour whole genome amplification reaction was performed by immersion in a whole genome amplification reaction reagent REPLI-g Single Cell Kit (QIAGEN) (S1000 thermocycler, Bio-Rad). The amplification reagent (REPLI-g Single Cell Kit) contained a component that neutralizes the potassium hydroxide solution (Buffer D2).

The gel capsules were stained with a fluorescent DNA intercalater. Gel capsules exhibiting fluorescence were counted by microscopy and flow cytometer and individually collected. As a result, a gel capsule exhibiting fluorescence was not observed from a dead bacteria model sample (Figure **2****)**.

The cell count and sequence information from live bacteria are obtained after distinguishing live bacteria from dead bacteria from a population containing both live bacteria and dead bacteria in the manner described above. Specifically, a gel capsule emitting fluorescence that indicates live bacteria among the gel capsules obtained in the manner described above are processed by the procedure described in Example 1, and some or all of the genome amplicon obtained from the gel capsules is sequenced. This allows sequence information derived from only live bacteria without dead bacteria to be selectively obtained, and the bacteria microbiota composition and total number to be evaluated only for live bacteria from the type and number of cells.

### Sequencing of a sample of single organism species after adding EMA

Gel capsules exhibiting fluorescence were collected from live bacteria model samples prepared by treating E. coli K12 live bacteria samples with EMA as described above. After dissolving gel capsules by heating individual collected gel capsules at 65°C (S1000 thermal cycler, Bio-Rad), secondary amplification was performed by MDA (REPLI-g Single Cell Kit, 150345, QIAGEN) within a well of each plate to prepare a library master plate comprising 10 µL DNA amplicon for each well.

A library was prepared using a Nextera XT DNA sample prep kit (Illumina, FC-131-1096) on the 12 samples described above. 2 × 75 bp paired-end reads were obtained by whole genome sequencing using Miseq (Illumina, SY-410-1003). Sequence data were assembled and Contig was prepared using SPAdes (Bankevich A et al., J Comput Biol. 2012 May; 19(5): 455-77. http://doi.org/10.1089/cmb.2012.002 1). For evaluation of the genome decoding rate (completion rate) and degree of contamination, analysis was performed under default conditions using CheckM (Parks et al., Genome Res. 2015. 25: 1043-1055, doi:10.1101/gr.186072.114). As a result, the mean completion rate was computed as 29.5% and the mean contamination rate was computed as 0.9% in EMA treated live bacteria models. The result suggests that genome data can be obtained by screening for live bacteria through EMA treatment in this method.

### Live bacteria sequencing from samples containing multiple organism species

Dead bacteria model samples were prepared from heating (95°C, 5 min) by using an NBRC microbe cocktail (Cell-Mock-001) (sample of mixture of 15 types of bacterial strains). Each of non-heated (live bacteria) samples and dead model samples was prepared. Cell pellets were suspended in 150 µL of phosphate buffered saline (PBS), treated using Biotium's PMA 20 mM in H₂O in accordance with the protocol, and treated by addition of EMA, permeation, and light irradiation. The suspension was then centrifuged for 3 minutes at 15000 × g to harvest the bacteria. After centrifuging and washing bacterial pellets twice with PBS, the pellets were suspended in PBS to obtain an E. coli suspension. The cell concentration in the prepared cell suspension was measured, and an ultralow melting point agarose was added so that the final concentration would be 1.5% to prepare an E. coli suspension used in the preparation of gel capsules (final cell concentration: 1.5 × 10³ cells/µL). Subsequent steps proceeded by the same procedure as Example 1.

The gel capsules were then stained with a fluorescent DNA intercalater. Gel capsules exhibiting fluorescence were counted by microscopy and flow cytometer and individually collected. As a result, a gel capsule exhibiting fluorescence was not observed from a dead bacteria model sample, while a gel capsule exhibiting fluorescence was observed from a live bacteria sample without PMA treatment and live bacteria sample with PMA treatment. After dissolving gel capsules by heating individual collected gel capsules at 65°C (S1000 thermal cycler, Bio-Rad), secondary amplification was performed by MDA (REPLI-g Single Cell Kit, 150345, QIAGEN) within a well of each plate to prepare a library master plate comprising 10 µL DNA amplicon for each well.

A library was prepared using a Nextera XT DNA sample prep kit (Illumina, FC-131-1096) on the samples described above. 2 × 75 bp paired-end reads were obtained by whole genome sequencing using Miseq (Illumina, SY-410-1003). Sequence data were assembled and Contig was prepared using SPAdes (Bankevich A et al., J Comput Biol. 2012 May; 19(5): 455-77. http://doi.org/10.1089/cmb.2012.002 1). For evaluation of the genome decoding rate (completion rate) and degree of contamination, analysis was performed under default conditions using CheckM (Parks et al., Genome Res. 2015. 25: 1043-1055, doi:10.1101/gr.186072.114). As a result of line annotation using 16S rRNA sequence or GT DB-tk, 11 types of bacteria genome data derived from Acinetobacter radioresistens, Staphylococcus epidermidis, Lactobacillus delbrueckii, Clostridium butyricum, Cutibacterium acnes, Bacillus subtilis, Escherichia coli, Corynebacterium striatum, Comamonas terrigena, and Parabacteroides distasonis were obtained from a microbe cocktail sample without PMA treatment (48 cell analysis), while 7 types of bacteria genome data from Staphylococcus epidermidis, Clostridium butyricum, Bacillus subtilis, Escherichia coli, Bacteroides uniformis, Parabacteroides distasonis, and Comamonas terrigena were obtained from a microbe cocktail sample with PMA treatment (96 cell analysis) (Table 1). Acinetobacter radioresistens, Lactobacillus delbrueckii, Cutibacterium acnes, and Corynebacterium striatum were eliminated at the single cell amplification genome library screening stage by PMA treatment, suggesting that many are dead bacteria in the microbe cocktail. Further, more Escherichia coli and Comamonas terrigena in particular were detected than others from the live bacteria sample. Thus, it was inferred that many cells were in the microbe cocktail as live bacteria.

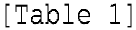

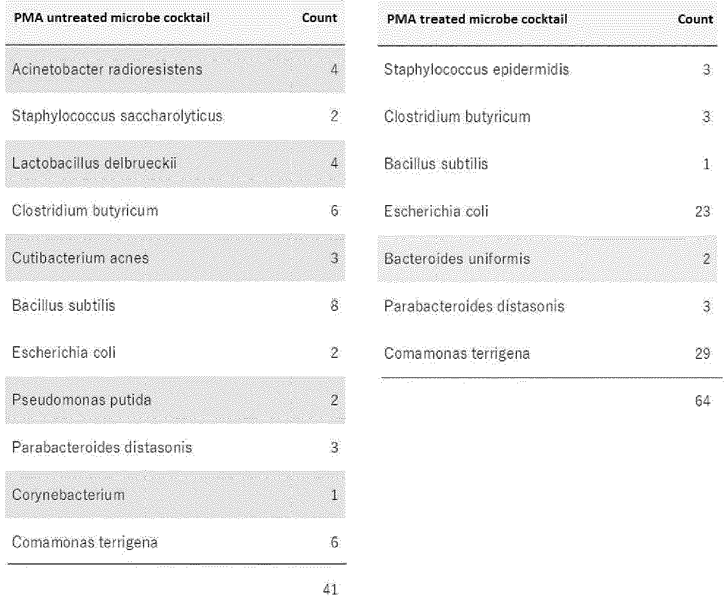

### (Example 3: Application Example)

### Example of evaluating quality of FMT specimen or microbial formulation

The technology of the invention can be applied to bacteria microbiota analysis or live bacteria count evaluation if the objective is to evaluate stability (activity or number) of microbes for stable supply or quality assurance in a transplant specimen in a population of microbes comprising one or more species of microbes such as a fecal microbiota transplant (FMT) specimen directed to prevention or treatment of a disease. For FMT specimens, bacteria microbiota varies by each specimen, with different bacteria subjected to study or bacteria count. Since the technology of the present disclosure can be applied even if the target species' component ratio or sequence information is unknown, the technology is suitable for bacteria microbiota analysis or live bacteria count evaluation. As a specific procedure, the FMT specimen samples are processed by the procedure described in Example 1, and some or all of the genome amplicon obtained from gel capsules is sequenced. This allows only sequence information derived from live bacteria to be selectively obtained, and the bacteria microbiota composition and total number to be evaluated only for live bacteria from the type and number of cells.

### Example of evaluating quality of microbial formulation

The technology of the invention can be applied to bacteria microbiota analysis or live bacteria count evaluation if the objective is to evaluate stability (activity or number) of microbes for stable supply or quality assurance in a formulation product in a population of microbes comprising one or more species of microbes such as a microbial formulation directed to prevention/treatment of a disease or promotion of health. In particular, an expected property may not be obtained due to mutation accumulation associated with long-term culture or manufacture or the like, but genome level analysis of diverse bacterial species can be performed with the technology of the present disclosure in terms of lot management between formulations. As a specific procedure, the samples are processed by the procedure described in Example 1, and some or all of the genome amplicon obtained from gel capsules is sequenced. This allows only sequence information derived from live bacteria to be selectively obtained, and the bacteria microbiota composition and total number to be evaluated only for live bacteria from the type and number of cells. Deterioration in quality due to contamination in the manufacturing step, accumulation of genetic mutations, or the like is evaluated by comparing the resulting genomic sequence with a reference genome.

### Example of use in inspection of food microbe

Sanitary indicative bacteria (contamination indicative bacteria) for evaluating sanitary quality of food products include common bacteria count (live bacteria count). However, official analytical methods that require culturing are time consuming and can be used for only specific bacteria. It is understood that the technology of the present disclosure can be advantageously applied if highly precise analysis is required. As a specific procedure, the samples are processed by the procedure described in Example 1, and some or all of the genome amplicon obtained from gel capsules is sequenced. This allows sequence information derived from live bacteria to be selectively obtained, and the number of live bacteria such as E. coli group/E. coli, Salmonella bacteria, Enterobacteriaceae bacteria, Staphylococcus aureus, or Vibrio parahaemolyticus, which are organisms currently targeted for food microbe inspections, is evaluated from the type and number thereof. The bacterial species is identified at the strain level by comparing the resulting genomic sequence with a reference genome.

### Example of use in investigation of live bacteria in an environment

It is understood that the technology of the present disclosure can be advantageously applied if the objective is to identify, to specifically detect, or to acquire genomic sequence information of multidrug resistant bacteria that are present as live bacteria in an environment, active bacteria with activity in various environments such as the gut, soil, or rhizosphere, microbes forming a biofilm, or bacteria understood to contribute to a host particularly in excellent soil, disease resistant rhizosphere, or the like. As a specific procedure, the samples are processed by the procedure described in Example 1, and some or all of the genome amplicon obtained from gel capsules is sequenced. This allows sequence information derived from live bacteria to be selectively obtained, and the bacteria microbiota composition and total number to be evaluated only for live bacteria from the type and number of cells, in addition to the novelty, contained genes or the like thereof to be evaluated from the genomic sequence information.

### (Example 4: When it is desirable to selectively acquire data on cells with a specific feature from diverse cells)

If the objective is to acquire genomic data for one or more specific types of microbes of interest to those skilled in the art among animal symbiotic microbes such as gut bacteria or marine/soil microbes, acquisition of unnecessary genetic sequence data and cost associated therewith can be reduced by checking the presence/absence of a gene fragment of target microbes in advance in a gel capsule comprising a polynucleotide or an amplified nucleic acid collected from a gel capsule. Examples of envisioned target of measurement include cases directed to comparative analysis of microbes of the same line (e.g., analysis of subspecies in a group of microbe lines associated with a disease or the like), search for microbes having a specific gene (e.g., secondary metabolite or enzyme produced by the microbes), individual selection and analysis of bacteria, archaea, fungi, other eukaryotes, or the like from multiple lines of organism species, and the like. In particular, specific detection of gut bacteria or oral bacteria serving a specific metabolic function from a gene, detection of subspecies in skin bacteria, and the like in order to evaluate the gut environment, oral cavity, or skin environment of the host human are envisioned.

### (Example 5: In case of coexistence of a large quantity of host DNA or the like)

When a sample to be analyzed is a stool, saliva, sputum, a liquid from swabbing the skin, oral cavity, nasal cavity, ear, genital, or the like, a surgical detergent, a tissue extract, or blood, and microbes contained in the sample are targeted for analysis, the sample contains many host animal derived cells, intracellular organelles, and nucleic acids. Polynucleotide amplification can be executed while some of them are encapsulated inside a gel capsule. For this reason, acquisition of unnecessary genetic sequence data comprising a host derived polynucleotide can be avoided and the cost associated therewith can be reduced by checking for the presence/absence of a genetic fragment of a target microbe or genetic fragment derived from a host in a gel capsule comprising an amplified polynucleotide in advance. Detection of microbes from a human derived sample such as search for pathogenic microbes from blood or sputum and analysis of symbiotic microbes inside a tissue are envisioned as a measurement example.

Alternatively, the invention can also be applied to integrated analysis of microbiota by proactively analyzing a host derived polynucleotide.

For example, it is known that a gut microbiota in the digestive tract and a host animal have a symbiotic relationship in which the host provides the digestive tract that is an anaerobic environment for colonization by the microbiota, while the gut microbiota affects the health of the host. Examples of significant effects on the health status of a host include production of nutrients, development of defense/immune system against infections, and the like. In other examples, inflammatory bowel diseases are diseases caused by an abnormality in the gut environmental factor including gut microbes together with genetic factors.

Such pathological conditions or the like require analysis of genetic information or the like of the host itself, comprehensive analysis of the gut microbiota including bacteria, viruses, and fungi, and elucidation of the mechanism of action on the physiological function of the host. The technology of the present disclosure can provide contribution thereto. Deeper analysis can be performed by analyzing the relationship between gut microbiota and the pathology of various diseases, including functional analysis around metabolites, genetic information or the like of the host itself, and the like.

While the gut bacteria and the organism are in a relationship of competing for nutrients in the intestinal tract, the gut bacteria also metabolize and decompose nutrients for the organism. To find what action a metabolite derived from gut bacteria exhibits, data from genetic analysis of the host side, metabolome analysis, biochemical analysis, or the like is acquired, the function of the gut bacteria is estimated from genomic sequence information of the bacteria, and various data such as the metabolite produced and metabolome is acquired. The relationship between the host and gut bacteria can be found by integrally analyzing data for both.

### (Example 6: Evaluation of genetic mutation in single cell unit)

The diversity of mutations on a genomic sequence can be evaluated in a single cell unit to track the occurrence or development of a mutant line or heterogeneity of genome of each microbe in the microbiota. Only data for genetic sequences targeted for analysis can be specifically acquired and the cost associated therewith can be reduced by selectively amplifying and detecting portion with a target genetic mutation of the target cell in a gel capsule comprising an amplified polynucleotide. This can be utilized in detection of a genetically mutant microbe or plasmid infection or the like.

### (Example 7: Evaluation of preservability of specific organism species)

If the objective is to prove or disprove that a drug, pesticide, food product, or the like comprises a specific organism species, the content thereof can be shown by detecting or screening for a specific organism from a gel capsule comprising an amplified polynucleotide by using the technology of the present disclosure. The degree of match, preservability, or the like with respect to a reference product can be evaluated at a genome level by analyzing the resulting genetic information in detail. Use in quality assurance of a microbial formulation or the like is envisioned.

As a specific procedure, the difference between a single organism derived genetic sequence and target genetic sequence is detected to identify a genetic mutation by performing, on a target gene, (1) homology search of the single organism derived genetic sequence using BLAST or hmmer or (2) mapping of single organism derived sequence data using BWA or bowtie2.

### (Example 8: Detection of specific organism species)

In some cases, a sample to be analyzed is a stool, saliva, sputum, a liquid from swabbing the skin, oral cavity, nasal cavity, ear, genital, or the like, a surgical detergent, a tissue extract, blood, or the like, and the presence of one or more specific species of microbes is detected, and drug efficacy or drug resistance is determined, or the ability to metabolize a food product is evaluated. The content of a specific organism can be shown by detecting or screening for the specific organism from a gel capsule comprising an amplified polynucleotide by using the technology of the present disclosure. At a genome level, a function can be estimated and preservability or the like can be evaluated by analyzing the resulting genetic information in detail.

If, for example, single cell derived sequence data for mouse gut microbes is obtained, microbe and groups of genes responsible for decomposition of food inulin were able to be identified and the function thereof was able to be estimated from the homology of known genes or prediction of the stereostructure based on the amino acid sequence by performing homology search on a single organism derived genetic sequence based on a gene that metabolizes dietary fiber inulin or a genetic marker that identifies Bacteroides species microbes. The proportion of the microbe species (content) in single cell derived sequence data for multiple gut microbes was able to be evaluated.

### (Example 9: Evaluation of soil bacteria)

If soil or seawater is the target sample to be analyzed, various specific organisms that inhabit soil or seawater can be detected or screened from a gel capsule comprising an amplified polynucleotide by using the technology of the present disclosure to identify the range of habitat thereof or show the quantity of inhabiting organisms. Further, the variety that is suitable for cultivation, animal husbandry, or aquaculture in such soil or seawater or the like can be evaluated at the genome level by analyzing the resulting genetic information in detail. Use in pesticide-free manufacturing method or the like using soil bacteria or seawater bacteria is also envisioned.

Specifically, 10 g of soil was collected (n = 3) in a 50 mL volume tube (2342-050, Iwaki) and thoroughly suspended by adding phosphate buffered saline (DPBS) (Dulbecco's Phosphate-Buffered Saline, 14190-144, Thermo Fisher Scientific) such that the total amount would be 40 mL. After leaving the post-suspension tube standing for 5 minutes on ice, the supernatant was transferred to a new 50 mL volume tube. The supernatant was filtered using a filter with a 5 µm pore size (SMWP04700, Sigma-Aldrich), and then centrifuged for 5 minutes at 10,000 × g (75004263, Thermo Fisher Scientific), and the supernatant was removed. Pellets were resuspended in 10 mL of PBS, dispensed to a 1.5 mL tube (MCT-150-C, Axygen) at 1 mL each, then centrifuged for 5 minutes at 10,000 × g to harvest soil bacteria. Bacterial pellets were combined in a single 1.5 mL tube and washed by centrifuging twice with PBS, and then suspended in PBS to obtain a cell suspension of soil bacteria. The cell concentration of the prepared cell suspension was measured (microscope: CKX41, OLYMPUS, bacteria counter A161, 2-5679-01, AS ONE), and ultralow melting point agarose (A5030-10G, Sigma-Aldrich) was added so that the final concentration would be 1.5% to prepare a soil bacteria suspension for use in gel capsule preparation (final cell concentration: 1.5 × 10³ cells/µL). Subsequently, analysis was performed by the same procedure as the approach described in Example 1 or 2 for evaluation.

### (Example 10: Analysis of composition of hydrosphere microbes)

In the experiment, 4L of seawater or freshwater was collected in a sterilized plastic container and filtered using a filter with a 5 µm pore size (SMWP04700, Sigma-Aldrich), then filtered using a filter with a 0.22 µm pore size (GSWP04700, Sigma-Aldrich) to trap bacterial fractions on the filter. The filter with a 0.22 µm pore size was placed in a 25 mL volume tube (2362-025, Iwaki) containing 10 mL of phosphate buffered saline (DPBS) (Dulbecco's Phosphate-Buffered Saline, 14190-144, Thermo Fisher Scientific) and thoroughly suspended. 10 mL of the cell suspension was dispensed to a 1.5 mL tube (MCT-150-C, Axygen) at 1 mL each, then centrifuged for 5 minutes at 10,000 × g to harvest seawater or freshwater derived bacteria. Bacterial pellets were combined in a single 1.5 mL tube and washed by centrifuging twice with PBS, and then suspended in PBS to obtain a cell suspension of seawater or freshwater derived bacteria. The cell concentration of the prepared cell suspension was measured (microscope: CKX41, OLYMPUS, bacteria counter A161, 2-5679-01, AS ONE), and ultralow melting point agarose (A5030-10G, Sigma-Aldrich) was added so that the final concentration would be 1.5% to prepare seawater or freshwater derived bacteria suspension for use in gel capsule preparation (final cell concentration: 1.5 × 10³ cells/µL). Subsequently, analysis was performed by the same procedure as the approach described in Example 1 or 2 for evaluation.

### [Industrial Applicability]

The present disclosure can be utilized in biological research, medicine, environment, healthcare, and other fields. If the objective is to prove or disprove that a drug, pesticide, food product, or the like contains specific organism species that are alive, the content thereof can be shown by detecting or screening for the specific organism from a gel capsule comprising an amplified polynucleotide by using the technology of the present disclosure. The degree of match, preservability, or the like with respect to a reference product can be evaluated at the genome level by analyzing the resulting genetic information in detail. Use in quality assurance of a microbial formulation or the like is envisioned. The present application claims priority to Japanese Patent Application No. 2019-85836 filed on April 26, 2019 and Japanese Patent Application No. 2019-201525 filed on November 6, 2019 with the Japan Patent Office. It is also understood that the content thereof should be incorporated herein by reference in the same manner as the contents are specifically described herein.

## Claims

1. A method of analyzing composition of a population of cells, comprising:
a step of providing gel capsules each encapsulating an individual cell of the population of cells being labeled with a labeling agent, wherein the labeling agent differentially labels a live cell and a dead cell as information on whether a cell is alive or dead for each individual cell in the population of cells;
a step of amplifying a nucleic acid derived from each individual cell in the population of cells;
a step of obtaining information on whether a cell is alive or dead for each individual cell in the population of cells; and
a step of analyzing an amplified nucleic acid derived from each individual cell in the population of cells.

2. The method of claim 1, wherein the step of providing gel capsules each encapsulating an individual cell of the population of cells comprises:
a step of adding the labeling agent to the population of cells; and
a step of individually encapsulating each of cells of the population of cells in the gel capsule.

3. The method of claim 1, wherein the step of providing gel capsules each encapsulating an individual cell of the population of cells comprises:
a step of individually encapsulating each of the cells of the population of cells in the gel capsule; and
a step of adding the labeling agent to the gel capsule.

4. The method of any one of claims 1 to 3, wherein the analysis of the amplified nucleic acid is performed on a nucleic acid derived from a live cell.

5. The method of any one of claims 1 to 4, wherein the labeling agent specifically labels a dead cell or specifically labels a live cell.

6. The method of any one of claims 1 to 5, further comprising a step of adding a nucleic acid degradation promoting agent for promoting degradation of a nucleic acid derived from a dead organism to the population of cells or the gel capsules.

7. The method of claim 6, wherein the nucleic acid degradation promoting agent is a topoisomerase inhibitor.

8. The method of claim 6, wherein the nucleic acid degradation promoting agent comprises one or more selected from Ciprofloxacin (CPFX), Camptothecin (CPT), Etoposide (ETP], and Amsacrine (m-AMSA).

9. The method of any one of claims 1 to 8, wherein the labeling agent is a substance that penetrates through a membrane of a dead cell.

10. The method of claim 9, wherein the labeling agent is a substance that penetrates through a membrane of a dead cell and binds to a nucleic acid.

11. The method of claim 10, wherein the labeling agent is a substance that inhibits amplification of a bound nucleic acid.

12. The method of any one of claims 1 to 11, wherein the labeling agent comprises one or more selected from ethidium monoazide (EMA), psoralen, 4'-aminomethyl-4,5'-dimethylisopsoralen [4'-AMDMIP) and propidium monoazide (PMA).

13. The method of any one of claims 1 to 12, wherein the information on whether a cell is alive or dead for each individual cell is obtained from the presence/absence of amplification of a nucleic acid derived from each individual cell.

14. The method of claim 13, wherein the presence/absence of amplification of a nucleic acid derived from each individual cell is detected using a nucleic acid binding fluorescent substance capable of detecting amplification of a nucleic acid.

15. The method of claim 14, wherein the nucleic acid binding fluorescent substance comprises one or more selected from EvaGreen, SYBR Green, SYBR Gold, SYTO9, SYTO10, Hoechst 33342, 4',6-diamidino-2-phenylindole, Acridine Orange (AO), Promidium iodide, and Ethidium homodimer-2.

16. The method of any one of claims 1 to 15, wherein the step of encapsulating cells of the population of cells each individually in the gel capsules comprises:
a step of individually encapsulating each of the cells of the population of cells in a liquid droplet; and
a step of gelating the liquid droplet to produce gel capsule.

17. The method of claim 16, wherein a suspension of the cells is allowed to flow in a microchannel, and the suspension is sheared with oil to prepare the liquid droplets encapsulating the cells.

18. The method of any one of claims 1 to 17, wherein the gel capsule is formed from agarose, acrylamide, PEG, gelatin, sodium alginate, Matrigel, collagen, or photocurable resin.

19. The method of any one of claims 1 to 18, wherein the gel capsules are hydrogel capsules.

20. The method of any one of claims 1 to 19, wherein the step of amplifying a nucleic acid derived from each individual cell in the population of cells comprises:
a step of immersing the gel capsules in one or more lysis reagents to lyse the cells, wherein a genomic DNA of the cells or a polynucleotide comprising said portion elutes out into the gel capsules and is retained in the gel capsules; and
a step of contacting the polynucleotide with an amplification reagent to amplify the polynucleotide in a gel capsule.

21. The method of any one of claims 1 to 20, further comprising a step of removing a contaminant from the gel capsules.

22. The method of claim 20, wherein at least one is selected as the lysis reagent from the group consisting of lysozyme, labiase, Yatalase, achromopeptidase, protease, nuclease, zymolyase, chitinase, lysostaphin, mutanolysin, sodium dodecyl sulfate, sodium lauryl sulfate, potassium hydroxide, sodium hydroxide, phenol, chloroform, guanidine hydrochloride, urea, 2-mercaptoethanol, dithiothreitol, TCEP-HCl, sodium cholate, sodium deoxycholate, Triton X-100, Triton X-114, NP-40, Brij-35, Brij-58, Tween 20, Tween 80, octyl glucoside, octyl thioglucoside, CHAPS, CHAPSO, dodecyl-p-D-maltoside, Nonidet P-40, and Zwittergent 3-12.

23. The method of any one of claims 1 to 22, further comprising a step of selecting a sample comprising the amplified nucleic acid to be analyzed from samples comprising the amplified nucleic acid from the each individual cell.

24. The method of any one of claims 1 to 23, wherein the analysis of the amplified nucleic acid comprises identifying a type of the each individual cell.

25. The method of any one of claims 1 to 24, wherein the analysis of the amplified nucleic acid comprises a step of detecting a nucleic acid having a specific sequence in samples comprising the amplified nucleic acid from the each individual cell.

26. The method of claim 25, wherein the step of detecting a nucleic acid having a specific sequence comprises amplifying a nucleic acid having a specific sequence and sequencing the nucleic acid.

27. The method of any one of claims 1 to 26, wherein the analysis of composition of a population of cells comprises identifying an absolute number of various cells in the population of cells.

28. The method of any one of claims 1 to 27, wherein the analysis of composition of a population of cells comprises identifying an absolute number of various cells in live cells in the population of cells.

29. The method of any one of claims 1 to 28, further comprising a step of obtaining genomic sequence data of the each individual cell from a sample comprising an amplified nucleic acid derived from each individual cell in the population of cells.

30. The method of any one of claims 1 to 29, wherein the population of cells is a microbiota.

31. The method of claim 30 for evaluating quality of an FMT specimen.

32. The method of claim 30 for evaluating sanitary quality of a food product.

33. The method of claim 30 for evaluating the presence of a pathogenic bacterium in an environment.

34. The method of any one of claims 1 to 29, wherein the population of cells is a microbial formulation.

35. The method of claim 34 for evaluating quality of a microbial formulation.

36. The method of any one of claims 1 to 29 for evaluating quality of a microbe derived animal feed, a fermented food product, or a microbe containing supplement.

37. The method of any one of claims 1 to 29 for profiling live bacteria in activated sludge.

38. The method of any one of claims 1 to 29 for evaluating the presence of a live microbe in an environment.

39. The method of any one of claims 1 to 29 for evaluating an effect or spectrum of an antibacterial drug.

40. The method of any one of claims 1 to 29 for evaluating a sterilization method.

41. The method of any one of claims 1 to 29 for profiling live bacteria in a biofilm.

42. A system or kit for analyzing composition of a population of cells, comprising:
a labeling agent for differentially labeling a live cell and a dead cell;
means for encapsulating the cells in gel capsules;
means for amplifying a nucleic acid; and
means for analyzing the nucleic acid.

43. A composition for use in a method of analyzing composition of a population of cells, comprising a labeling agent for differentially labeling a live cell and a dead cell, wherein the method comprises: a step of obtaining information on whether a cell is alive or dead for each individual cell in the population of cells; and a step of analyzing an amplified nucleic acid derived from each individual cell in the population of cells.

44. A composition for use in a method of encapsulating cells of a population of cells each individually in gel capsules for analysis, comprising a labeling agent for differentially labeling a live cell and a dead cell.
